# EUROPÄISCHE PATENTSCHRIFT

(11) **EP 2 091 866 B1**
(45) Veröffentlichungstag und Bekanntmachung des Hinweises auf die Patenterteilung: **10.07.2013**
(21) Anmeldenummer: 07847970.6
(22) Anmeldetag: 07.12.2007
(51) Int. Cl.: C01B 21/22, B01D 53/14, B01D 53/56, C07C 45/28, C07C 49/00

(54) **VERFAHREN ZUR ISOLIERUNG VON N2O**
METHOD FOR ISOLATING N2O
PROCÉDÉ D'ISOLEMENT DE N2O

(30) Priorität: 11.12.2006 EP 06125807
(43) Veröffentlichungstag der Anmeldung: 26.08.2009
(73) Patentinhaber: BASF SE, 67056 Ludwigshafen (DE)
(72) Erfinder: BAUMANN, Dieter, 67346 Speyer (DE); RÖSSLER, Beatrice, 67256 Weisenheim am Sand (DE); TELES, Joaquim Henrique, 67166 Otterstadt (DE)
(86) Internationale Anmeldenummer: PCT/EP2007/063510
(87) Internationale Veröffentlichungsnummer: WO 2008/071632

(56) Entgegenhaltungen:
- WO-A-2006/032502
- DE-A1- 2 040 219
- US-A- 4 154 806
- DATABASE CA [Online] CHEMICAL ABSTRACTS SERVICE, COLUMBUS, OHIO, US; MIKODA, JIRI: "Highly concentrated dinitrogen monoxide" XP002481314 gefunden im STN Database accession no. 82:5746 & CS 153 889 B1 29. März 1974 (1974-03-29)
- DATABASE CA [Online] CHEMICAL ABSTRACTS SERVICE, COLUMBUS, OHIO, US; MIKODA, JIRI: "Separation of dinitrogen monoxide [from gas mixtures]" XP002481315 gefunden im STN Database accession no. 85:96530 & CS 161 507 B1 (CZECH.) 10. Juni 1975 (1975-06-10)

## Beschreibung

Die vorliegende Erfindung betrifft ein Verfahren zur Reinigung eines Gasgemisches G-0 enthaltend Distickstoffmonoxid, mindestens umfassend das Inkontaktbringen des Gasgemisches G-0 mit einem Lösungsmittelgemisch (I) mindestens enthaltend 50 Gew.-% Wasser bezogen auf das gesamte Lösungsmittelgemisch (I), wobei der pH-Wert des Lösungsmittelgemischs (I) im Bereich von 3,5 bis 8,0 liegt, die Desorption eines Gasgemisches G-1 aus einer Zusammensetzung (A), das Inkontaktbringen des Gasgemisches G-1 mit einem Lösungsmittelgemisch (II) mindestens enthaltend 50 Gew.-% Wasser bezogen auf das gesamte Lösungsmittelgemisch (II), wobei der pH-Wert des Lösungsmittelgemischs (II) im Bereich von 2,0 bis 8,0 liegt, und die Desorption eines Gasgemisches G-2 aus einer Zusammensetzung (B), wobei sich der pH-Wert jeweils auf eine Messung mit einer kalibrierten und temperaturkompensierten Glaselektrode bezieht, sowie die Verwendung von nach einem erfindungsgemäßen Verfahren erhaltenen Gasgemischen als Oxidationsmittel für Olefine.

Aus dem Stand der Technik sind verschiedene Herstellungsverfahren und Reinigungsverfahren für Distickstoffmonoxid bekannt. Es ist ebenfalls bekannt, dass Distickstoffmonoxid beispielsweise als Oxidationsmittel für Olefine eingesetzt werden kann.

So offenbart die WO 98/25698 ein Verfahren zur Herstellung von Distickstoffmonoxid durch katalytische Partialoxidation von NH₃ mit Sauerstoff. Dabei wird gemäß der WO 98/25698 ein Katalysator aus Manganoxid, Bismutoxid und Aluminiumoxid eingesetzt, der mit hoher Selektivität zu Distickstoffmonoxid führt. Ein ähnliches Katalysatorsystem wird auch in einer wissenschaftlichen Arbeit näher beschrieben (Noskov et al., Chem. Eng. J. 91 (2003) 235-242). Gemäß der US 5,849,257 wird ebenfalls ein Verfahren zur Herstellung von Distickstoffmonoxid durch Oxidation von Ammoniak offenbart. Dabei findet die Oxidation in Gegenwart eines Kupfer-Manganoxid Katalysators statt.

Gemäß dem in der WO 00/01654 offenbarten Verfahren wird Distickstoffmonoxid hergestellt, indem ein Gasstrom enthaltend NOₓ und Ammoniak reduziert wird.

Die Oxidation einer olefinischen Verbindung zu einem Aldehyd oder einem Keton mittels Distickstoffmonoxid ist beispielsweise beschrieben in der GB 649,680 oder der dazu äquivalenten US 2,636,898. In beiden Schriften wird ganz allgemein offenbart, dass die Oxidation prinzipiell in Anwesenheit eines geeigneten Oxidationskatalysators erfolgen kann.

In den neueren wissenschaftlichen Artikeln von G. I. Panov et al., "Non-Catalytic Liquid Phase Oxidation of Alkenes with Nitrous Oxide. 1. Oxidation of Cyclohexene to Cyclohexanone", React. Kinet. Catal. Lett. Vol. 76, No. 2 (2002) S. 401-405, und K. A. Dubkov et al., "Non-Catalytic Liquid Phase Oxidation of Alkenes with Nitrous Oxide. 2. Oxidation of Cyclopentene to Cyclopentanone", React. Kinet. Catal. Lett. Vol. 77, No. 1 (2002) S. 197-205 werden ebenfalls Oxidationen von olefinischen Verbindungen mit Distickstoffmonoxid beschrieben. Auch ein wissenschaftlicher Artikel "Liquid Phase Oxidation of Alkenes with Nitrous Oxide to Carbonyl Compounds" von E. V. Starokon et al. in Adv. Synth. Catal. 2004, 346, 268 - 274 beinhaltet eine mechanistische Studie der Oxidation von Alkenen mit Distickstoffmonoxid in flüssiger Phase.

Die Synthese von Carbonylverbindungen aus Alkenen mit Distickstoffmonoxid wird auch in verschiedenen internationalen Patentanmeldungen beschrieben. So offenbart die WO 03/078370 ein Verfahren zur Herstellung von Carbonylverbindungen aus aliphatischen Alkenen mit Distickstoffmonoxid. Die Umsetzung wird bei Temperaturen im Bereich von 20 bis 350°C und Drücken von 0,01 bis 100 atm durchgeführt. Die WO 03/078374 offenbart ein entsprechendes Verfahren zur Herstellung von Cyclohexanon. Gemäß der WO 03/078372 werden cyclische Ketone mit 4 bis 5 C-Atomen hergestellt. Gemäß der WO 03/078375 werden unter diesen Verfahrensbedingungen cyclische Ketone aus cyclischen Alkenen mit 7 bis 20 C-Atomen hergestellt. WO 03/078371 offenbart ein Verfahren zur Herstellung substituierter Ketone aus substituierten Alkenen. WO 04/000777 offenbart ein Verfahren zur Umsetzung von Di- und Polyalkenen mit Distickstoffmonoxid zu den entsprechenden Carbonylverbindungen. Die Reinigung von Distickstoffmonoxid wird in diesen Schriften nicht erwähnt.

Es ist ebenfalls bekannt, dass Abgasströme enthaltend Distickstoffmonoxid für weitere Umsetzungen eingesetzt werden können. Distickstoffmonoxid fällt als unerwünschtes Nebenprodukt bei verschiedenen chemischen Prozessen an, insbesondere bei Oxidationen mit Salpetersäure und dort ganz besonders bei der Oxidation von Cyclohexanon und/oder Cyclohexanol zu Adipinsäure. Andere Beispiele für Verfahren, bei denen Distickstoffmonoxid als unerwünschtes Nebenprodukt anfällt, sind die Oxidation von Cyclododecanon und/oder Cyclododecanol mit Salpetersäure zu Dodecandicarbonsäure und die Partialoxidation von NH₃ zu NO.

So offenbaren die WO 2005/030690, die WO 2005/030689 und die WO 2004/096745 Verfahren zur Oxidation von Olefinen mit Distickstoffmonoxid, nämlich die Oxidation von Cyclododecatrien, von Cyclododecen und von Cyclopenten. Alle drei Anmeldungen offenbaren, dass neben anderen Distickstoffmonoxidquellen auch Abgasströme eingesetzt werden können, die beispielsweise durch destillative Methoden aufgereinigt werden können, bevor sie als Oxidationsmittel eingesetzt werden.

Sowohl bei der Herstellung von Distickstoffmonoxid als auch bei der Verwendung von Abgasströmen fällt N₂O zunächst als verdünntes gasförmiges Gemisch mit anderen Komponenten an. Diese Komponenten lassen sich unterteilen in solche, die für spezielle Anwendungen störend wirken, und solche, die sich inert verhalten. Für den Einsatz als Oxidationsmittel sind als solche störend wirkenden Gase unter anderem NOₓ oder beispielsweise Sauerstoff (O₂) zu nennen. Der Begriff "NOₓ", wie er im Rahmen der vorliegenden Erfindung verstanden wird, bezeichnet sämtliche Verbindungen NₐO_{b}, wobei a 1 oder 2 ist und b eine Zahl von 1 bis 6, außer N₂O. Statt dem Begriff "NOₓ" wird im Rahmen der vorliegenden Erfindung auch der Begriff "Stickoxide" verwendet. Als störende Nebenkomponenten sind auch NH₃ und organischen Säuren zu nennen.

Für spezielle Anwendungen ist es nötig, das eingesetzte Distickstoffmonoxid vor der Umsetzung zu reinigen. Beispielsweise für die Verwendung von Distickstoffmonoxid als Oxidationsmittel ist es nötig, störende Nebenkomponenten wie Sauerstoff oder Stickoxide NOₓ abzutrennen.

Verfahren zur Abtrennung von NOₓ sind grundsätzlich aus dem Stand der Technik bekannt. Eine Übersicht gibt beispielsweise M. Thiemann et. al in Ullmann's Encyclopedia, 6th Edition, 2000, Electronic Edition, Kapitel "Nitric Acid, Nitrous Acid, and Nitrogen Oxides", Abschnitt 1.4.2.3.

Die Anmeldung WO 00/73202 beschreibt eine Methode, wie man NOₓ und O₂ aus einem N₂O-haltigen Gasstrom entfernt werden kann. Das NOₓ wird durch katalytische Reduktion mit NH₃ entfernt und Sauerstoff durch katalytische Reduktion mit Wasserstoff oder anderen Reduktionsmitteln. Diese Methode hat aber den Nachteil, dass das Produkt mit NH₃ kontaminiert wird. Eine starke Abreicherung von Sauerstoff ist nur möglich, wenn ein Verlust an N₂O, von beispielsweise 3 bis 5% der ursprünglich enthaltenen Menge, in Kauf genommen wird.

Für spezielle Anwendungen kann es notwendig sein, auch die inerten Verbindungen abzutrennen, da sie die gewünschte Umsetzung mit N₂O durch Verdünnung verlangsamen können. Der Begriff "Inertgas", wie er im Rahmen der vorliegenden Erfindung verwendet wird, bezeichnet ein Gas, das sich hinsichtlich der Umsetzung von N₂O mit einem Olefin inert verhält, also unter den Bedingungen der Umsetzung von Olefinen mit N₂O weder mit den Olefinen noch mit N₂O reagieren. Als Inertgase sind beispielsweise Stickstoff, Kohlendioxid, Argon, Methan, Ethan und Propan zu nennen. Die Inertgase können aber die Raum-Zeit-Ausbeute senken, so dass eine Abreicherung ebenfalls vorteilhaft sein kann. Es kann aber ebenfalls vorteilhaft sein, ein Gasgemisch zu erhalten, das noch Inertgase wie beispielsweise Kohlenstoffdioxid enthält, und das dann direkt in eine weitere Umsetzung eingesetzt werden kann.

In DE 27 32 267 A1 wird beispielsweise ein Verfahren zur Reinigung von Distickstoffmonoxid offenbart, wobei zunächst Stickstoffoxid, Stickstoffdioxid, Kohlenstoffdioxid und Wasser abgetrennt werden und anschließend das Gasgemisch durch Kompression auf 40 bis 300 bar und Kühlung auf 0 bis -88°C verflüssigt wird. Aus diesem verflüssigten Gasgemisch wird dann Distickstoffmonoxid abgetrennt. Diese Methode erreicht zwar eine Reinigung und Aufkonzentrierung des N₂O, ist aber aufgrund des geforderten hohen Drucks (60 bar), der tiefen Temperaturen (-85°C) und den damit verbundenen hohen Investitionen, wirtschaftlich unattraktiv.

In US 4,177,645 wird ein Verfahren zur Abtrennung von Distickstoffmonoxid aus Abgasströmen offenbart, das ebenfalls eine Vorreinigung und eine Tieftemperaturdestillation umfasst. Die Anmeldung EP 1 076 217 A1 beschreibt ebenfalls eine Methode zur Entfernung von leichtsiedenden Verunreinigungen aus N₂O durch Tieftemperaturdestillation.

Auch US 6,505,482, US 6,370,911 und US 6,387,161 offenbaren Verfahren zur Reinigung von Distickstoffmonoxid, bei dem jeweils eine Tieftemperaturdestillation in einer speziellen Anlage durchgeführt wird.

Eine Tieftemperaturdestillation erfordert durch die hohen Drücke und tiefen Temperaturen jedoch apparativ einen hohen Aufwand, der die Reinigung des Distickstoffmonoxids mit einem derartigen Verfahren aufwändig und kostenintensiv macht. Besonders störend ist hierbei die Tatsache, dass bei Normaldruck der Schmelzpunkt von N₂O nur 3K unterhalb des Siedepunkts liegt. Daher müssen hohe Drücke angewandt werden.

DT 20 40 219 offenbart ein Herstellungsverfahren für Distickstoffmonoxid, wobei das erhaltene Distickstoffmonoxid nach der Synthese konzentriert und gereinigt wird. Dabei wird gemäß der DT 20 40 219 zunächst Distickstoffmonoxid durch Oxidation von Ammoniak hergestellt. Das hergestellte Distickstoffmonoxid wird gereinigt, indem die oxidierten Gase separiert werden, und durch Absorption unter hohem Druck, der eine Desorption unter vermindertem Druck folgt, konzentriert. Nebenkomponenten werden beispielsweise durch Behandlung mit einer Alkalilösung in einem Waschturm entfernt. Als Lösungsmittel für die Absorption des Gasgemisches wird gemäß DT 20 40 219 Wasser verwendet.

Mit dem in DT 20 40 219 offenbarten Verfahren ist eine Trennung der verschiedenen Stickoxide möglich, das Verfahren erfordert jedoch den Einsatz von großen Lösungsmittelmengen und/oder hoher Drücke für die Absorption. Gleichzeitig ist für das gemäß DT 20 40 219 offenbarte Verfahren zur Abtrennung von weiteren störenden Komponenten ein weiterer Waschturm nötig.

In WO 2006/032502 wird ein Verfahren zur Reinigung eines Gasgemisches enthaltend Distickstoffmonoxid offenbart, das mindestens eine Absorption des Gasgemisches in einem organischen Lösungsmittel und anschließende Desorption des Gasgemisches aus dem beladenen organischen Lösungsmittel sowie das Einstellen des Gehalts an Stickoxiden NOₓ in dem Gasgemisch auf höchstens 0,5 Vol.-%, bezogen auf das Gesamtvolumen des Gasgemisches umfasst. In WO 2006/032502 wird auch offenbart, dass das Verfahren mehrere Absorptions- und Desorptionsschritte umfassen kann. In WO 2006/032502 werden nur organische Lösungsmittel als Absorptionsmedium offenbart.

Die DE 10 2005 055588.5 betrifft ein Verfahren zur Reinigung eines Gasgemisches G-0 enthaltend Distickstoffmonoxid, mindestens umfassend die Absorption des Gasgemisches G-0 in einem organischen Lösungsmittel, anschließende Desorption eines Gasgemisches G-1 aus dem beladenen organischen Lösungsmittel, Absorption des Gasgemisches G-1 in Wasser und anschließende Desorption eines Gasgemisches G-2 aus dem beladenen Wasser, sowie die Verwendung eines gereinigten Gasgemischs enthaltend Distickstoffmonoxid, erhältlich nach einem derartigen Verfahren als Oxidationsmittel für Olefine.

Ausgehend von diesem Stand der Technik lag eine Aufgabe der vorliegenden Erfindung darin, ein sicherheitstechnisch unbedenkliches Verfahren bereitzustellen, mit dem Distickstoffmonoxid-haltige Ströme effektiv und kostengünstig gereinigt und aufkonzentriert werden können. Derart aufgereinigtes Distickstoffmonoxid wird insbesondere als Oxidationsmittel benötigt.

Eine weitere Aufgabe der vorliegenden Erfindung war es, Verfahren zur Herstellung von Gasgemischen bereitzustellen, die ohne weitere Behandlung oder Zusatz anderer Inertisierungsmittel als Oxidationsmittel eingesetzt werden können.

Erfindungsgemäß wird diese Aufgabe gelöst durch ein Verfahren zur Reinigung eines Gasgemisches G-0 enthaltend Distickstoffmonoxid und NOₓ mindestens umfassend die folgenden Schritte:
A1 Inkontaktbringen des Gasgemisches G-0 mit einem Lösungsmittelgemisch (I) mindestens enthaltend 50 Gew.-% Wasser bezogen auf das gesamte Lösungsmittelgemisch (I), wobei der pH-Wert des Lösungsmittelgemischs (I) im Bereich von 3,5 bis 8,0 liegt, unter Erhalt einer Zusammensetzung (A);
A2 Desorption eines Gasgemisches G-1 aus der Zusammensetzung (A) unter Erhalt eines Lösungsmittelgemischs (I');
B1 Inkontaktbringen des Gasgemisches G-1 mit einem Lösungsmittelgemisch (II) mindestens enthaltend 50 Gew.-% Wasser bezogen auf das gesamte Lösungsmittelgemisch (II), wobei der pH-Wert des Lösungsmittelgemischs (II) im Bereich von 2,0 bis 8,0 liegt, unter Erhalt einer Zusammensetzung (B);
B2 Desorption eines Gasgemisches G-2 aus der Zusammensetzung (B) unter Erhalt eines Lösungsmittelgemischs (II');
wobei sich der pH-Wert jeweils auf eine Messung mit einer Glaselektrode, insbesondere mit einer kalibrierten und temperaturkompensierten Glaselektrode, bezieht
wobei der pH-Wert des Lösungsmittelgemischs (I) oder der pH-Wert des Lösungsmittelgemischs (II) oder der pH-Wert des Lösungsmittelgemischs (I) und der pH-Wert des Lösungsmittelgemischs (II) durch Zugabe einer Base eingestellt wird.

Im Kontext dieser Anmeldung wird der pH-Wert mit einer kommerziell erhältlichen Glaselektrode gemessen, die vorher gegen Puffer mit bekanntem pH-Wert kalibriert wurde. Alle Angaben von pH-Werten beziehen sich auf eine Messung mit einer kalibrierten und temperaturkompensierten Glaselektrode. Falls die Kalibriertemperatur von der Messtemperatur abweicht wird eine Temperaturkompensierung verwendet. Diese Definition und dieses Vorgehen entspricht der derzeit gültigen IUPAC Empfehlung (R.P.Buck et al., Pure Appl. Chem. (2002) 74(11), S. 2169-2200 und dort insbesondere Abschnitt 11).

Das erfindungsgemäße Verfahren hat unter anderem den Vorteil, dass neben den störenden Komponenten teilweise auch die inerten Komponenten abgetrennt werden. Das erfindungsgemäß gereinigte Distickstoffmonoxid wird also gleichzeitig aufkonzentriert. Durch das erfindungsgemäße Verfahren verbleibt jedoch Kohlenstoffdioxid, das inertisierend wirkt, zumindest teilweise im Gasgemisch G-2, so dass das Gasgemisch G-2 direkt sicherheitstechnisch unbedenklich als Oxidationsmittel ohne Zusatz weiterer Inertgase eingesetzt werden kann.

Dabei hat das erfindungsgemäße Verfahren darüber hinaus den Vorteil, dass das Lösungsmittelgemisch (I) oder (II) mindestens enthaltend 50 Gew.-% Wasser, jeweils bezogen auf das gesamte Lösungsmittelgemisch (I) bzw. (II), für Schritt A1 bzw. Schritt B1 eingesetzt wird. Damit wird vermieden, dass Distickstoffmonoxid als starkes Oxidationsmittel in höheren Konzentrationen mit einem organischen Lösungsmittel in Kontakt gebracht wird, was zu einem hohen apparativen Aufwand und hohen Kosten führen würde. Durch die erfindungsgemäße Verwendung von Lösungsmittelgemisch (I) bzw. (II) mindestens enthaltend 50 Gew.-% Wasser, bezogen auf das gesamte Lösungsmittelgemisch (I) bzw. (II), bei dem Inkontaktbringen und der Desorption gemäß Schritt A1 und A2 bzw. Schritt B1 und B2 wird darüber hinaus vermieden, dass das Gasgemisch mit organischem Lösungsmittel kontaminiert wird, was zu weiteren Reinigungsschritten führen könnte.

Somit ist es mit dem erfindungsgemäßen Verfahren möglich, ein sicherheitstechnisch unbedenkliches Oxidationsmittel als Gasgemisch bereitzustellen.

Durch die erfindungsgemäße Auswahl des pH-Wertes von Lösungsmittelgemisch (I) und Lösungsmittelgemisch (II) wird eine fast vollständige Abreicherung von NOₓ erzielt. Dadurch wird eine separate Abtrennung von NOₓ, beispielsweise mittels DeNOx oder SCR-DeNOx überflüssig. Dadurch gibt es bei dem erfindungsgemäßen Verfahren beispielsweise auch keine Gefahr der Kontamination vom Produktstrom mit NH₃ der für die DeNOx-Stufe als Reduktionsmittel verwendet wird.

Durch die erfindungsgemäße gezielte Auswahl des pH-Wertes des Lösungsmittelgemischs (I) und des Lösungsmittelgemischs (II) kann insbesondere eine gute Abreicherung von NOₓ mit nur einer minimalen Änderung des Kohlenstoffdioxid-Gehaltes erreicht werden.

Das erfindungsgemäß eingesetzte Lösungsmittelgemisch (I) und (II) weisen bei dem erfindungsgemäßen pH-Wert eine hohe Selektivität für die gewünschten Komponenten, insbesondere Distickstoffmonoxid und Kohlenstoffdioxid, auf. Gleichzeitig ist die absolute Löslichkeit von Distickstoffmonoxid in dem erfindungsgemäß eingesetzten Lösungsmittelgemisch (I) bzw. (II) ausreichend, um eine Aufkonzentrierung zu erreichen. Dabei hat das erfindungsgemäß eingesetzte Lösungsmittelgemisch (I) bzw. (II) den Vorteil, dass auch unter Druck in Gegenwart von konzentriertem Distickstoffmonoxid keine sicherheitstechnischen Probleme auftreten.

Erfindungsgemäß weist das Gasgemisch G-1 einen höheren Gehalt an Distickstoffmonoxid auf als das Gasgemisch G-0. Das Gasgemisch G-2 weist erfindungsgemäß wiederum einen höheren Gehalt an Distickstoffmonoxid auf als das Gasgemisch G-1.

Das erfindungsgemäß gereinigte Gasgemisch G-2 kann insbesondere in flüssiger Form vorteilhaft als Oxidationsmittel eingesetzt werden. Vorteilhafterweise enthält das Gasgemisch G-2 neben Distickstoffmonoxid auch Kohlenstoffdioxid. CO₂ wirkt inertisierend für Gasgemische enthaltend Distickstoffmonoxid und gewährleistet einen sicherheitstechnisch unbedenklichen Betrieb bei der Aufbereitung und insbesondere bei der Lagerung und weiteren Verwendung des Gasgemischs G-2 enthaltend Distickstoffmonoxid. Es wurde gefunden, dass bei der Anwesenheit von CO₂ als Inertgas in Gasgemischen enthaltend N₂O im Vergleich zu anderen Inertgasen deutlich geringere Mengen Kohlenstoffdioxid benötigt werden, um die Selbstzerfallfähigkeit von Distickstoffmonoxid zu unterbinden. Damit reichen geringe Mengen an CO₂ zur Inertisierung des Gasgemischs G-2 aus.

Das erfindungsgemäße Verfahren zur Reinigung eines Gasgemisches G-0 enthaltend Distickstoffmonoxid umfasst die Schritte A1, A2, B1 und B2.

Dabei wird zunächst gemäß Schritt A1 das Gasgemisch G-0 mit einem Lösungsmittelgemisch (I) mindestens enthaltend 50 Gew.-% Wasser bezogen auf das gesamte Lösungsmittelgemisch (I), wobei der pH-Wert des Lösungsmittelgemischs (I) im Bereich von 3,5 bis 8,0 liegt, in Kontakt gebracht, wobei eine Zusammensetzung (A) erhalten wird. Beim Inkontaktbringen des Gasgemischs G-0 mit dem Lösungsmittelgemisch (I) wird erfindungsgemäß das Gasgemisch G-0 zumindest teilweise im Lösungsmittelgemisch (I) absorbiert. Erfindungsgemäß liegt der pH-Wert des Lösungsmittelgemischs (I) im Bereich von 3,5 bis 8,0, so dass insbesondere eine selektive Absorption einzelner im Gasgemisch G-0 enthaltener Gase erfolgt, wobei andere Bestandteile des Gasgemischs G-0 nicht oder nur in geringerem Ausmaß absorbiert werden.

Der pH-Wert kann erfindungsgemäß ohne weitere Maßnahmen in diesem Bereich liegen. Es ist jedoch erfindungsgemäß ebenso möglich, den pH-Wert in diesem Bereich durch geeignete Maßnahmen einzustellen, beispielsweise durch Zugabe von Lauge. Bevorzugt wird der pH-Wert des Lösungsmittelgemischs (I) erfindungsgemäß im Bereich von 3,5 bis 8,0 durch geeignete Maßnahmen eingestellt. Besonders bevorzugt wird der pH-Wert des Lösungsmittelgemischs (I) erfindungsgemäß im Bereich von 3,5 bis 8,0 durch Zugabe mindestens einer Lauge eingestellt.

Gemäß Schritt A1 wird eine Zusammensetzung (A) erhalten, die das Lösungsmittelgemisch (I) sowie absorbierte Bestandteile des Gasgemischs G-0 und gegebenenfalls Reaktionsprodukte der absorbierten Bestandteile enthält. Gleichzeitig wird bei dem Inkontaktbringen gemäß Schritt A1 ein an den absorbierten Bestandteilen verarmtes Gasgemisch erhalten, das erfindungsgemäß vorzugsweise nicht weiter umgesetzt wird.

Die Zusammensetzung (A) wird erfindungsgemäß in Schritt A2 weiter behandelt. Gemäß Schritt A2 erfolgt eine Desorption eines Gasgemisches G-1 aus der Zusammensetzung (A) unter Erhalt eines Lösungsmittelgemischs (I'). Dabei werden die zuvor absorbierten Gase teilweise oder vollständig desorbiert. Erfindungsgemäß erfolgt eine möglichst vollständige Desorption von Distickstoffmonoxid, wobei beispielsweise andere Stickoxide NOₓ nicht desorbiert werden. Das Lösungsmittelgemisch (I') kann insbesondere Salze enthalten. Das Gasgemisch G-1 enthält erfindungsgemäß insbesondere Distickstoffmonoxid, Stickstoff und Kohlenstoffdioxid.

Das Gasgemisch G-0 enthält erfindungsgemäß beispielsweise Distickstoffmonoxid, Stickstoff, Sauerstoff, Kohlenstoffdioxid, Argon und Kohlenstoffmonoxid.

Erfindungsgemäß wird das Gasgemisch G-1 gemäß Schritt B1 weiter behandelt. Dabei wird das Gasgemisch G-1 mit einem Lösungsmittelgemisch (II) mindestens enthaltend 50 Gew.-% Wasser bezogen auf das gesamte Lösungsmittelgemisch (II), wobei der pH-Wert des Lösungsmittelgemischs (II) im Bereich von 2,0 bis 8,0 liegt, in Kontakt gebracht, wobei eine Zusammensetzung (B) erhalten wird. Beim Inkontaktbringen des Gasgemischs G-1 mit dem Lösungsmittelgemisch (II) wird erfindungsgemäß das Gasgemisch G-1 zumindest teilweise im Lösungsmittelgemisch (II) absorbiert.

Erfindungsgemäß liegt der pH-Wert des Lösungsmittelgemischs (II) im Bereich von 2,0 bis 8,0, so dass insbesondere eine selektive Absorption einzelner im Gasgemisch G-1 enthaltener Gase erfolgt, wobei andere Bestandteile des Gasgemischs G-1 nicht oder nur in geringerem Ausmaß absorbiert werden. Gemäß Schritt B1 wird eine Zusammensetzung (B) erhalten, die das Lösungsmittelgemisch (II) sowie absorbierte Bestandteile des Gasgemischs G-1 und gegebenenfalls Reaktionsprodukte der absorbierten Bestandteile enthält. Gleichzeitig wird bei dem Inkontaktbringen gemäß Schritt B1 ein an den absorbierten Bestandteilen verarmtes Gasgemisch erhalten, das erfindungsgemäß vorzugsweise nicht weiter umgesetzt wird.

Die Zusammensetzung (B) wird erfindungsgemäß in Schritt B2 weiter behandelt. Gemäß Schritt B2 erfolgt eine Desorption eines Gasgemisches G-2 aus der Zusammensetzung (B) unter Erhalt eines Lösungsmittelgemischs (II'). Dabei werden die zuvor absorbierten Gase teilweise oder vollständig desorbiert. Erfindungsgemäß erfolgt eine möglichst vollständige Desorption von Distickstoffmonoxid, wobei beispielsweise andere Stickoxide NOₓ nicht desorbiert werden. Das Lösungsmittelgemisch (II') kann insbesondere Salze enthalten.

Erfindungsgemäß kann das eingesetzte Gasgemisch G-0 enthaltend Distickstoffmonoxid und NOₓ grundsätzlich aus jeder beliebigen Quelle stammen.

Der Begriff "Gasgemisch", wie er im Rahmen der vorliegenden Erfindung verwendet wird, bezeichnet ein Gemisch aus zwei oder mehr Verbindungen, die sich bei Umgebungsdruck und Umgebungstemperatur im gasförmigen Zustand befinden. Bei veränderter Temperatur oder verändertem Druck kann das Gasgemisch auch in einem anderen Aggregatzustand vorliegen; beispielsweise flüssig, und wird im Rahmen der vorliegenden Erfindung weiter als Gasgemisch bezeichnet.

Im Rahmen der vorliegenden Erfindung wird die Zusammensetzung der Gasgemische oder der verflüssigten Gasgemische in Vol.-% angegeben. Dabei beziehen sich die Angaben auf die Zusammensetzung der Gasgemische bei Umgebungsdruck und Umgebungstemperatur.

Grundsätzlich kann die Zusammensetzung der Gemische im Rahmen der vorliegenden Erfindung auf jede dem Fachmann bekannte Weise bestimmt werden. Die Zusammensetzung der Gasgemische wird im Rahmen der vorliegenden Erfindung vorzugsweise gaschromatographisch bestimmt. Sie kann jedoch auch mittels UV-Spektroskopie, IR-Spektroskopie oder nasschemisch bestimmt werden.

Wird ein Gasgemisch G-0 eingesetzt, so ist dessen Gehalt an Distickstoffmonoxid im Wesentlichen beliebig, solange gewährleistet ist, dass die erfindungsgemäße Reinigung möglich ist.

Die N₂O-haltige Gasgemische, die für dieses Verfahren als Gasgemisch G-0 eingesetzt werden, haben in der Regel einen N₂O-Gehalt zwischen 2 und 80 Vol.-% N₂O. Es enthält ferner beispielsweise 2 bis 21 Vol.-% O₂ und bis zu 30 Vol.-% NOₓ als unerwünschte Komponenten. Ferner kann es noch in wechselnden Mengen N₂, H₂, CO₂, CO, H₂O, NH₃ enthalten, in Spuren können auch noch organische Verbindungen enthalten sein. Beispielsweise kann das Gasgemisch G-0 auch 9 bis 13 Vol.-% N₂ und bis zu 5,5 Vol.-% NH₃ enthalten. Dabei ergibt die Summe der Komponenten des Gasgemischs G-0 100 Vol.-%.

Gemäß einer bevorzugten Ausführungsform des erfindungsgemäßen Verfahrens wird ein mindestens 3 Vol.-% Distickstoffmonoxid enthaltendes Gasgemisch G-0 eingesetzt, wobei wiederum bevorzugt Gemische mit einem Distickstoffmonoxid-Gehalt im Bereich von 4 bis 60 Vol.-%, weiter bevorzugt im Bereich von 5 bis 25 Vol.-% und insbesondere bevorzugt im Bereich von 8 bis 14 Vol.-% eingesetzt werden.

Gemäß einer bevorzugten Ausführungsform der vorliegenden Erfindung ist das Gasgemisch G-0 enthaltend Distickstoffmonoxid und NOₓ mindestens ein Distickstoffmonoxid enthaltendes Abgas eines chemischen Verfahrens. Im Rahmen der vorliegenden Erfindung sind auch Ausführungsformen umfasst, bei denen mindestens zwei Stickstoffmonoxid enthaltende Abgase einer einzigen Anlage als Gasgemisch enthaltend Distickstoffmonoxid dienen. Ebenso sind Ausführungsformen umfasst, bei denen mindestens ein Distickstoffmonoxid enthaltendes Abgas einer Anlage und mindestens ein weiteres Distickstoffmonoxid enthaltendes Abgas mindestens einer weiteren Anlage als Gasgemisch enthaltend Distickstoffmonoxid dienen.

Demgemäß betrifft die vorliegende Erfindung auch ein wie oben beschriebenes Verfahren, wobei das Gasgemisch enthaltend Distickstoffmonoxid mindestens ein Distickstoffmonoxid enthaltendes Abgas mindestens eines industriellen Verfahrens ist.

Der Begriff "Gasgemisch enthaltend Distickstoffmonoxid" bezeichnet im Rahmen der vorliegenden Erfindung sowohl Ausführungsformen, in denen das genannte Abgas in unmodifizierter Form dem erfindungsgemäßen Reinigungsverfahren unterworfen wird, als auch Ausführungsformen, in denen mindestens eines der genannten Abgase einer Modifikation unterworfen wird.

Der Begriff "Modifikation", wie er in diesem Zusammenhang im Rahmen der vorliegenden Erfindung verwendet wird, bezeichnet jedes geeignete Verfahren, mit dem die chemische Zusammensetzung eines Gasgemisches verändert wird. Demgemäß umfasst der Begriff "Modifikation" unter anderem Ausführungsformen, in denen ein Distickstoffmonoxid enthaltendes Abgas bezüglich des Distickstoffmonoxid-Gehaltes gemäß mindestens eines geeigneten Verfahrens aufkonzentriert wird. Vorzugsweise wird das Abgas keiner Modifikation unterworfen.

Gemäß einer weiteren Ausführungsform kann die chemische Zusammensetzung eines Abgases auch durch Zusatz von reinem Distickstoffmonoxid zu dem Abgas verändert werden.

Das eingesetzte Gasgemisch G-0 enthaltend N₂O und NOₓ kann beispielsweise ein Abgas aus einem industriellen Verfahren sein. Vorzugsweise stammt es aus einem Abgas einer Anlage zur Herstellung von Carbonsäuren durch Oxidation von Alkoholen oder Ketonen mit Salpetersäure, wie z.B. aus einer Adipinsäure- oder DodecandicarbonsäureAnlage, aus dem Abgas einer Salpetersäure-Anlage die die obigen Abgasströme als Edukt einsetzt, aus dem Abgas einer Anlage zur Partialoxidation von NH₃ oder aus dem Abgas einer Anlage die die darin erzeugten Gasgemische einsetzt, wie z.B. einer Hydroxylamin-Anlage.

Erfindungsgemäß kann auch ein Gemisch verschiedener Abgase eingesetzt werden.

Gemäß einer weiter bevorzugten Ausführungsform der vorliegenden Erfindung stammt das mindestens eine Distickstoffmonoxid enthaltende Abgas aus einer Adipinsäureanlage, einer Dodecandicarbonsäureanlage, einer Hydroxylaminanlage und/oder einer Salpetersäureanlage, wobei letztere wiederum bevorzugt mit mindestens einem Abgas einer Adipinsäureanlage, einer Dodecandicarbonsäureanlage oder einer Hydroxylamin-anlage betrieben wird.

Gemäß einer bevorzugten Ausführungsform wird der Abgasstrom einer Adipinsäureanlage eingesetzt, bei der durch Oxidation von Cyclohexanol/Cyclohexanon-Gemischen mit Salpetersäure pro Mol gebildeter Adipinsäure im Allgemeinen 0,8 bis 1,0 mol N₂O gebildet werden. Wie beispielsweise in A. K. Uriarte et al., Stud. Surf. Sci. Catal. 130 (2000) S. 743-748 beschrieben, enthalten die Abgase von Adipinsäureanlagen in unterschiedlichen Konzentrationen noch weitere Bestandteile wie unter anderem Stickstoff, Sauerstoff, Kohlendioxid, Kohlenmonoxid, Stickoxide, Wasser und flüchtige organische Verbindungen.

Bei der obenstehend erwähnten Dodecandicarbonsäureanlage handelt es sich um den im Wesentlichen identischen Anlagentyp.

Eine beispielsweise typische Zusammensetzung eines Abgases einer Adipinsäureanlage oder einer Dodecandicarbonsäureanlage ist in der folgenden Tabelle wiedergegeben:

| Komponente | Konzentrationen / Gew.-% |
|---|---|
| NO_{X} | 19 - 25 |
| N₂O | 20 - 28 |
| N₂ | 30 - 40 |
| O₂ | 7 - 10 |
| CO₂ | 2 - 3 |
| H₂O | ∼ 7 |

Der Abgasstrom einer Adipinsäureanlage oder einer Dodecandicarbonsäureanlage kann direkt in dem erfindungsgemäßen Verfahren eingesetzt werden.

Gemäß einer ebenfalls bevorzugten Ausführungsform wird der Abgasstrom einer Salpetersäureanlage eingesetzt, die ganz oder teilweise mit Distickstoffmonoxid und Stickoxide enthaltenden Abgasen aus anderen Verfahren gespeist wird. In derartigen Salpetersäureanlagen werden Stickoxide adsorbiert und zum größten Teil zu Salpetersäure umgesetzt, während Distickstoffmonoxid nicht umgesetzt wird. Beispielsweise kann eine derartige Salpetersäureanlage durch Stickoxide, die durch gezielte Verbrennung von Ammoniak hergestellt werden, und durch Abgase einer Adipinsäureanlage und/oder durch Abgase einer Dodecandicarbonsäureanlage gespeist werden. Ebenso ist es möglich, eine derartige Salpetersäureanlage allein durch Abgase einer Adipinsäureanlage und/oder durch Abgase einer Dodecandicarbonsäureanlage zu speisen.

Die Abgase von derartigen Salpetersäureanlagen enthalten grundsätzlich in unterschiedlichen Konzentrationen noch weitere Bestandteile wie unter anderem Stickstoff, Sauerstoff, Kohlendioxid, Kohlenmonoxid, Stickoxide, Wasser und flüchtige organische Verbindungen.

Eine beispielsweise typische Zusammensetzung eines Abgases einer derartigen Salpetersäureanlage ist in der folgenden Tabelle wiedergegeben:

| Komponente | Konzentrationen / Gew.-% |
|---|---|
| NOₓ | < 0,1 |
| N₂O | 4 - 36 |
| N₂ | 57 - 86 |
| O₂ | 3 - 9 |
| CO₂ | 1 - 4 |
| H₂O | ∼ 0,6 |

Der Abgasstrom einer Salpetersäureanlage kann direkt in dem erfindungsgemäßen Verfahren eingesetzt werden.

Gemäß einer ebenfalls bevorzugten Ausführungsform des erfindungsgemäßen Verfahrens wird der Abgasstrom einer Hydroxylaminanlage eingesetzt, bei der beispielsweise zunächst Ammoniak mit Luft oder Sauerstoff zu NO oxidiert wird, wobei kleine Mengen an Distickstoffmonoxid als Nebenprodukt gebildet werden. Das NO wird anschließend mit Wasserstoff zu Hydroxylamin hydriert. Nachdem Distickstoffmonoxid unter den Hydrierbedingungen inert ist, reichert es sich im Wasserstoffkreis an. In bevorzugten Verfahrensführungen enthält der Purge-Strom einer Hydroxylaminanlage Distickstoffmonoxid im Bereich von 9 bis 13 Vol.-% in Wasserstoff. Dieser Purge-Strom kann als solcher zur erfindungsgemäßen Reinigung eingesetzt werden. Ebenso ist es möglich, diesen Strom bezüglich des Distickstoffmonoxid-Gehaltes, wie oben beschrieben, geeignet aufzukonzentrieren.

Demgemäß betrifft die vorliegende Erfindung auch ein wie oben beschriebenes Verfahren, wobei das Gasgemisch G-0 das Abgas einer Adipinsäureanlage und/oder einer Dodecandicarbonsäureanlage und/oder einer Hydroxylaminanlage und/oder einer mit dem Abgas einer Adipinsäureanlage und/oder einer Dodecandicarbonsäureanlage und/oder einer Hydroxylaminanlage betriebenen Salpetersäureanlage ist.

Ebenso kann im Rahmen des erfindungsgemäßen Verfahrens Distickstoffmonoxid zum Einsatz im Verfahren gezielt hergestellt werden. Bevorzugt wird dabei unter anderem die Herstellung über die thermische Zersetzung von NH₄NO₃, wie dies beispielsweise in US 3,656,899 beschrieben ist, deren diesbezüglicher Inhalt durch Bezugnahme in den Kontext der vorliegenden Anmeldung vollumfänglich aufgenommen wird. Ebenfalls bevorzugt wird weiter die Herstellung über die katalytische Oxidation von Ammoniak, wie dies beispielsweise in US 5,849,257 oder in WO 98/25698 beschrieben ist, deren diesbezüglicher Inhalt durch Bezugnahme in den Kontext der vorliegenden Anmeldung vollumfänglich aufgenommen wird.

Die N₂O-Konzentrierung erfolgt erfindungsgemäß durch ein Inkontaktbringen des Gasgemischs G-0 mit dem Lösungsmittelgemisch (I) und damit eine erste Absorption von N₂O und damit vorzugsweise aufgrund der ähnlichen physikalischen Eigenschaften auch von CO₂ aus dem Gasgemisch G-0 in dem Lösungsmittelgemisch (I) und anschließender Desorption des Gasgemischs G-1 aus der Zusammensetzung (A) gemäß Schritt A1 und A2.

Erfindungsgemäß liegt der pH-Wert des Lösungsmittelgemischs (I) bei dem Inkontaktbringen gemäß Schritt A1 im Bereich von 3,5 bis 8,0. Bei diesem pH-Wert erfolgt erfindungsgemäß eine gute Absorption von Distickstoffmonoxid und Kohlenstoffdioxid im Lösungsmittelgemisch (I), wobei andere Gase, die im Gasgemisch G-0 enthalten sein können, nicht oder wenig absorbiert werden. Bevorzugt liegt der pH-Wert in einem Bereich von 5,0 bis 5,7 besonders bevorzugt in einem Bereich von 6,0 bis 7,0.

Dabei wird erfindungsgemäß der pH-Wert vor oder während des Inkontaktbringens gemäß Schritt A1 des erfindungsgemäßen Verfahrens gemessen und dann beispielsweise der pH-Wert durch geeignete Maßnahmen eingestellt. Ebenso ist es erfindungsgemäß möglich, dass keine Maßnahmen nötig sind, um den pH-Wert einzustellen.

Grundsätzlich kann der pH-Wert erfindungsgemäß durch alle dem Fachmann bekannten Maßnahmen eingestellt werden. Geeignete Maßnahmen zum Einstellen des pH-Werts sind beispielsweise Zugabe einer Lauge oder Zugabe von weiterem Lösungsmittel.

Beispielsweise wird der pH-Wert des Lösungsmittelgemischs (I) vor oder nach dem Inkontaktbringen gemäß Schritt A1 gemessen und der pH-Wert im erfindungsgemäßen Bereich durch geeignete Maßnahmen eingestellt. Die Messung des pH-Werts kann dabei erfindungsgemäß kontinuierlich oder diskontinuierlich erfolgen.

Bei dem Inkontaktbringen gemäß Schritt A1 erfolgt erfindungsgemäß eine Absorption, wobei eine Zusammensetzung (A) und ein an den absorbierten Gasen verarmter Gasstrom erhalten wird. Die Zusammensetzung (A) weist dabei erfindungsgemäß einen anderen pH-Wert auf, als das Lösungsmittelgemisch (I).

Erfindungsgemäß enthält die Zusammensetzung (A) beispielsweise 90,0 bis 99,9999 Gew.-% Wasser, insbesondere 95,0 bis 99,999 Gew.-%, bevorzugt 98,0 bis 99,99 Gew.-% Wasser; beispielsweise 0,01 bis 0,25 Gew.-% Distickstoffmonoxid, insbesondere 0,05 bis 0,2 Gew.-%, bevorzugt 0,1 bis 0,15 Gew.-% Distickstoffmonoxid; beispielsweise 0,0001 bis 0,1 Gew.-% Kohlenstoffdioxid, insbesondere 0,001 bis 0,05 Gew.-% Kohlenstoffdioxid; beispielsweise 0,0001 bis 0,1 Gew.-% Stickstoff, insbesondere 0,001 bis 0,05 Gew.-% Stickstoff; beispielsweise 0,05 bis 1,5 Gew.-% Natriumnitrit, insbesondere 0,1 bis 1,0 Gew.-%, bevorzugt 0,25 bis 0,75 Gew.-% Natriumnitrit; beispielsweise 0,05 bis 1,5 Gew.-% Natriumnitrat, insbesondere 0,1 bis 1,0 Gew.-%, bevorzugt 0,25 bis 0,75 Gew.-% Natriumnitrat; ; beispielsweise 0,0001 bis 0,1 Gew.-% Natriumhydrogencarbonat, insbesondere 0,001 bis 0,05 Gew.-% Natriumhydrogencarbonat; sowie Spuren von Sauerstoff und Argon. Dabei ergibt die Summe der Komponenten der Zusammensetzung (A) 100 Gew.-%.

Erfindungsgemäß enthält der verarmte Gasstrom beispielsweise 0,1 bis 2,0 Vol.-% Argon, insbesondere 0,25 bis 1,5 Vol.-%, bevorzugt 0,5 bis 1,0 Vol.-% Argon; beispielsweise 1,0 bis 10 Vol.-% Sauerstoff, insbesondere 2,5 bis 7,5 Vol.-%, bevorzugt 4,0 bis 6,0 Vol.-% Sauerstoff; beispielsweise 1,0 bis 10 Vol.-% Distickstoffmonoxid, insbesondere 2,5 bis 7,5 Vol.-%, bevorzugt 4,0 bis 6,0 Vol.-% Distickstoffmonoxid; beispielsweise 70 bis 99,9 Vol.-% Stickstoff, insbesondere 75 bis 95 Vol.-%, bevorzugt 80 bis 90 Vol.-% Stickstoff; beispielsweise 0,01 bis 0,5 Vol.-% Kohlenstoffmonoxid, insbesondere 0,05 bis 0,25 Vol.-%, bevorzugt 0,08 bis 0,1 Vol.-% Kohlenstoffmonoxid; beispielsweise 0,1 bis 1,5 Vol.-% Kohlenstoffdioxid, insbesondere 0,25 bis 1,0 Vol.-%, bevorzugt 0,5 bis 0,75 Vol.-% Kohlenstoffdioxid; beispielsweise 0,1 bis 1,5 Vol.-% Wasser, insbesondere 0,25 bis 1,0 Vol.-%, bevorzugt 0,5 bis 0,75 Vol.-% Wasser. Dabei ergibt die Summe der Komponenten des Gasstroms 100 Vol.-%.

Vorzugsweise wird Schritt A1 des erfindungsgemäßen Verfahrens kontinuierlich durchgeführt. Das heißt im Rahmen der vorliegenden Erfindung, dass kontinuierlich das Lösungsmittelgemisch (I) und das Gasgemisch G-0 in Kontakt gebracht werden, wobei sich kontinuierlich die Zusammensetzung (A) und der verarmte Gasstrom bilden.

Erfindungsgemäß werden bei dem Inkontaktbringen gemäß Schritt A1 vorzugsweise Distickstoffmonoxid und Kohlenstoffdioxid absorbiert. Erfindungsgemäß können beispielsweise auch Stickstoff, Sauerstoff und Argon absorbiert werden. Auch Stickoxide NOₓ werden gemäß Schritt A1 absorbiert.

Das erfindungsgemäße Verfahren umfasst weiter einen Schritt A2, bei dem ein Gasgemisch G-1 aus der Zusammensetzung (A) unter Erhalt eines Lösungsmittelgemischs (I') desorbiert wird.

Dabei werden gemäß Schritt A2 vorzugsweise Distickstoffmonoxid und Kohlenstoffdioxid aus der Zusammensetzung (A) desorbiert.

Beispielsweise für den Fall, dass im Erfindungsgemäßen Verfahren der pH-Wert durch Zugabe einer Lauge, insbesondere Natronlauge, eingestellt wird, enthält das Lösungsmittelgemisch ()') erfindungsgemäß beispielsweise 90,0 bis 99,9999 Gew.-% Wasser, insbesondere 95,0 bis 99,999 Gew.-%, bevorzugt 98,0 bis 99,99 Gew.-% Wasser; beispielsweise 0,001 bis 0,1 Gew.-% Distickstoffmonoxid, beispielsweise 0,05 bis 1,5 Gew.-% Natriumnitrit, insbesondere 0,1 bis 1,0 Gew.-%, bevorzugt 0,25 bis 0,75 Gew.-% Natriumnitrit; beispielsweise 0,05 bis 1,5 Gew.-% Natriumnitrat, insbesondere 0,1 bis 1,0 Gew.-%, bevorzugt 0,25 bis 0,75 Gew.-% Natriumnitrat; ; beispielsweise 0,0001 bis 0,1 Gew.-% Natriumhydrogencarbonat, insbesondere 0,001 bis 0,05 Gew.-% Natriumhydrogencarbonat. Das Lösungsmittelgemisch (I') kann darüber hinaus auch weitere Verbindungen enthalten. Dabei ergibt die Summe der Komponenten der Zusammensetzung (A) 100 Gew.-%.

Das erfindungsgemäß eingesetzte Lösungsmittelgemisch (I) mindestens enthaltend 50 Gew.-% Wasser, bezogen auf das gesamte Lösungsmittelgemisch (I), enthält beispielsweise 50 bis 100 Gew.-% Wasser, vorzugsweise 60 bis 99,999 Gew.-% Wasser, insbesondere 70 bis 99 Gew.-% Wasser, besonders bevorzugt 80 bis 98 Gew.-% Wasser, beispielsweise 90 bis 95 Gew.-% Wasser. Gemäß einer Ausführungsform enthält das Lösungsmittelgemisch (I) mindestens 80 Gew.-% Wasser, jeweils bezogen auf das gesamte Lösungsmittelgemisch (I).

Erfindungsgemäß kann das Lösungsmittelgemisch neben Wasser auch andere polare mit Wasser mischbare Lösungsmittel enthalten, beispielsweise Glykole. Vorzugsweise enthält das Lösungsmittelgemisch (I) keine weiteren polaren Lösungsmittel. Darüber hinaus kann das Lösungsmittelgemisch (I) neben Wasser auch gelöste Salze enthalten, beispielsweise Salze der Alkali- oder Erdalkalimetalle, insbesondere Hydroxide, Hydrogencarbonate, Carbonate, Nitrate, Nitrite, Sulfate, Hydrogenphosphate oder Phosphate.

Erfindungsgemäß ist der Gehalt an Salzen im Lösungsmittelgemisch (I) kleiner als 5 Gew.-%, bevorzugt kleiner als 2,5 Gew.-%, insbesondere kleiner als 1,0 Gew.-%, besonders bevorzugt kleiner als 0,5 Gew.-%. Der Gehalt an Salzen im Lösungsmittelgemisch (I) beträgt beispielsweise 0,0001 bis 5 Gew.-%, bevorzugt 0,001 bis 2,5 Gew.-%, insbesondere 0,01 bis 1,0 Gew.-%.

Bei der Desorption gemäß Schritt A2 wird ein Gasgemisch G-1 und ein Lösungsmittelgemisch (I') erhalten.

Im Rahmen der vorliegenden Erfindung hat das Gasgemisch G-1 beispielsweise einen Gehalt an N₂O von 40 bis 80 Vol.-%, vorzugsweise von 45 bis 75 Vol.-%, insbesondere von 50 bis 65 Vol.-%, besonders bevorzugt beispielsweise 51 Vol.-%, 52 Vol.-%, 53 Vol.-% 54 Vol.-%, 55 Vol.-%, 56 Vol.-%, 57 Vol.-%, 58 Vol.-%, 59 Vol.-%, 60 Vol.%, 61 Vol.-%, 62 Vol.-%, 63 Vol.-%, 64 Vol.-% oder 65 Vol.-%.

Das Gasgemisch G-1 hat beispielsweise einen Gehalt an CO₂ von 5 bis 15 Vol.-%, vorzugsweise von 6 bis 12 Vol.-%, besonders bevorzugt beispielsweise 7 Vol.-%, 9 Vol.-%, 10 Vol.-% oder 11 Vol.-%. Gleichzeitig hat das Gasgemisch G-1 beispielsweise einen Gehalt an O₂ von 1,0 bis 4,0 Vol.-%, vorzugsweise von 1,5 bis 3,5 Vol.-%, besonders bevorzugt 2,5 bis 3.1 Vol.-%, beispielsweise 2,6 Vol.-%, 2,7 Vol.-%, 2,8 Vol.-%, 2,9 Vol.-% oder 3,0 Vol.-%. Darüber hinaus kann das Gasgemisch G-1 noch 20 bis 40 Vol.-% N₂ enthalten, vorzugsweise 20 bis 35 Vol.-%, sowie weitere Komponenten, beispielsweise Stickoxide. NOₓ kann dabei beispielsweise in einer Menge von 0 bis 0,1 Vol.-% enthalten sein, vorzugsweise 0,0001 bis 0,01 Vol.-%, besonders bevorzugt 0,0002 bis 0,05 Vol.-%. Dabei ergibt die Summe der Komponenten des Gasgemischs G-1 100 Vol.-%. Das Gasgemisch G-1 kann darüber hinaus noch 0 bis 10 Vol.-% Wasser enthalten, insbesondere 2 bis 8 Vol.-%, bevorzugt 4 bis 6 Vol.-% Wasser.

Gemäß Schritt B1 und B2 erfolgt ein Inkontaktbringen des Gasgemischs G-1 mit einem geeigneten Lösungsmittelgemisch (II) und eine anschließende Desorption des Gasgemischs G-2.

Das erfindungsgemäße Verfahren weist weiter einen Schritt B1 und einen Schritt B2 auf. Erfindungsgemäß liegt der pH-Wert des Lösungsmittelgemischs (II) bei dem Inkontaktbringen gemäß Schritt B1 im Bereich von 2,0 bis 8,0. Bei diesem pH-Wert erfolgt erfindungsgemäß eine gute Absorption von Distickstoffmonoxid und Kohlenstoffdioxid im Lösungsmittelgemisch (II), wobei andere Gase, die im Gasgemisch G-1 enthalten sein können, nicht oder wenig absorbiert werden. Bevorzugt liegt der pH-Wert in einem Bereich von 2,5 bis 8,0, insbesondere 3,5 bis 8,0, weiter bevorzugt 5,0 bis 5,7 besonders bevorzugt in einem Bereich von 6,0 bis 7,0.

Der pH-Wert kann erfindungsgemäß ohne weitere Maßnahmen in diesem Bereich liegen. Es ist jedoch erfindungsgemäß ebenso möglich, den pH-Wert in diesem Bereich durch geeignete Maßnahmen einzustellen, beispielsweise durch Zugabe von Lauge.

Dabei kann erfindungsgemäß der pH-Wert vor oder während des Inkontaktbringens gemäß Schritt B1 des erfindungsgemäßen Verfahrens gemessen werden und dann der pH-Wert gegebenenfalls durch geeignete Maßnahmen eingestellt werden. Die Messung des pH-Werts kann dabei erfindungsgemäß kontinuierlich oder diskontinuierlich erfolgen.

Bevorzugt wird der pH-Wert des Lösungsmittelgemischs (II) vor dem Inkontaktbringen gemäß Schritt B1 gemessen und der pH-Wert im erfindungsgemäßen Bereich eingestellt.

Bei dem Inkontaktbringen gemäß Schritt B1 erfolgt erfindungsgemäß eine Absorption, wobei eine Zusammensetzung (B) und ein an den absorbierten Gasen verarmter Gasstrom erhalten wird. Die Zusammensetzung (B) weist dabei erfindungsgemäß einen anderen pH-Wert auf, als das Lösungsmittelgemisch (II).

Erfingdungsgemäß enthält die Zusammensetzung (B) beispielsweise 90,0 bis 99,9999 Gew.-% Wasser, insbesondere 95,0 bis 99,999 Gew.-%, bevorzugt 98,0 bis 99,99 Gew.-% Wasser; beispielsweise 0,01 bis 2,5 Gew.-% Distickstoffmonoxid, insbesondere 0,1 bis 1,5 Gew.-%, bevorzugt 0,5 bis 1,0 Gew.-% Distickstoffmonoxid; beispielsweise 0,001 bis 0,5 Gew.-% Kohlenstoffdioxid, insbesondere 0,01 bis 0,25 Gew.-% Kohlenstoffdioxid; beispielsweise 0,0001 bis 0,1 Gew.-% Stickstoff, insbesondere 0,001 bis 0,05 Gew.-% Stickstoff; sowie Spuren von Sauerstoff und Argon. Dabei ergibt die Summe der Komponenten der Zusammensetzung (B) 100 Gew.-%.

Vorzugsweise wird Schritt B1 des erfindungsgemäßen Verfahrens kontinuierlich durchgeführt. Das heißt im Rahmen der vorliegenden Erfindung, dass kontinuierlich das Lösungsmittelgemisch (II) und das Gasgemisch G-1 in Kontakt gebracht werden, wobei sich kontinuierlich die Zusammensetzung (B) und der verarmte Gasstrom bilden.

Vorzugsweise werden die Schritte A1 und B1 des erfindungsgemäßen Verfahrens kontinuierlich durchgeführt.

Erfindungsgemäß werden bei dem Inkontaktbringen gemäß Schritt B1 vorzugsweise Distickstoffmonoxid und Kohlenstoffdioxid absorbiert. Auch im Gasgemisch G-1 verbleibende Stickoxide NOₓ werden gemäß Schritt B1 absorbiert.

Vom eintretenden Gasstrom werden erfindungsgemäß vorzugsweise 60 bis 80 % absorbiert.

Das erfindungsgemäße Verfahren umfasst weiter einen Schritt B2, bei dem ein Gasgemisch G-2 aus der Zusammensetzung (B) unter Erhalt eines Lösungsmittelgemischs (II') desorbiert wird.

Dabei werden gemäß Schritt B2 vorzugsweise Distickstoffmonoxid und Kohlenstoffdioxid aus der Zusammensetzung (B) desorbiert. Das Lösungsmittelgemisch (II') kann erfindungsgemäß einen anderen pH-Wert auf als die Zusammensetzung (B) oder das Lösungsmittelgemisch (II) aufweisen. Ebenso ist es erfindungsgemäß möglich, dass das Lösungsmittelgemisch (II'), die Zusammensetzung (B) und das Lösungsmittelgemisch (II) denselben pH-Wert aufweisen, bzw. einen pH-Wert, der im selben Bereich liegt.

Sofern der pH-Wert des Lösungsmittelgemischs (I) und des Lösungsmittelgemischs (II) eingestellt werden, können der pH-Wert des Lösungsmittelgemischs (I) und des Lösungsmittelgemischs (II) unabhängig voneinander eingestellt werden. Erfindungsgemäß ist es auch möglich, dass nur der pH-Wert des Lösungsmittelgemischs (I) oder des Lösungsmittelgemischs (II) eingestellt werden. Der pH-Wert des Lösungsmittelgemischs (I) und des Lösungsmittelgemischs (II) können jedoch erfindungsgemäß auch im gleichen Bereich eingestellt werden.

Unabhängig von dem erfindungsgemäß eingesetzten Lösungsmittelgemisch (I) enthält das erfindungsgemäß eingesetzte Lösungsmittelgemisch (II) mindestens enthaltend 50 Gew.-% Wasser beispielsweise 50 bis 100 Gew.-% Wasser, vorzugsweise 60 bis 99,999 Gew.-% Wasser, insbesondere 70 bis 99 Gew.-% Wasser, besonders bevorzugt 80 bis 98 Gew.-% Wasser, beispielsweise 90 bis 95 Gew.-% Wasser. Gemäß einer Ausführungsform enthält das Lösungsmittelgemisch (II) mindestens 80 Gew.-% Wasser, jeweils bezogen auf das gesamte Lösungsmittelgemisch (II).

Daher betrifft die vorliegende Erfindung auch ein wie zuvor beschriebenes Verfahren zur Reinigung eines Gasgemischs G-0 enthaltend Distickstoffmonoxid, wobei das Lösungsmittelgemisch (I) oder das Lösungsmittelgemisch (II) oder das Lösungsmittelgemisch (I) und das Lösungsmittelgemisch (II) mindestens 80 Gew.-% Wasser enthält, jeweils bezogen auf das gesamte Lösungsmittelgemisch (I) oder (II).

Erfindungsgemäß kann das Lösungsmittelgemisch neben Wasser auch andere polare mit Wasser mischbare Lösungsmittel enthalten, beispielsweise Glykole. Vorzugsweise enthält das Lösungsmittelgemisch (II) keine weiteren polaren Lösungsmittel. Darüber hinaus kann das Lösungsmittelgemisch (II) neben Wasser auch gelöste Salze enthalten, beispielsweise Salze der Alkali- oder Erdalkalimetalle, insbesondere Hydroxide, Hydrogencarbonate, Carbonate, Nitrate, Nitrite, Sulfate, Hydrogenphosphate oder Phosphate.

Erfindungsgemäß ist der Gehalt an Salzen im Lösungsmittelgemisch (I) kleiner als 5 Gew.-%, bevorzugt kleiner als 2,5 Gew.-%, insbesondere kleiner als 1,0 Gew.-%, besonders bevorzugt kleiner als 0,5 Gew.-%. Der Gehalt an Salzen im Lösungsmittelgemisch (I) beträgt beispielsweise 0,0001 bis 5 Gew.-%, bevorzugt 0,001 bis 2,5 Gew.-%, insbesondere 0,01 bis 1,0 Gew.-%.

Daher betrifft die vorliegende Erfindung auch ein wie zuvor beschriebenes Verfahren zur Reinigung eines Gasgemischs G-0 enthaltend Distickstoffmonoxid und NOₓ, wobei das Lösungsmittelgemisch (I) oder das Lösungsmittelgemisch (II) oder das Lösungsmittelgemisch (I) und das Lösungsmittelgemisch (II) weniger als 10 Gew.-% Salze enthält, jeweils bezogen auf das gesamte Lösungsmittelgemisch (I) oder (II).

Der pH-Wert des Lösungsmittelgemischs (I) liegt erfindungsgemäß vorzugsweise in einem Bereich von 3,5 und 8,0, besonders bevorzugt im Bereich 5,0 bis 7,5 und ganz besonders bevorzugt im Bereich 6,0 bis 7,0. Der pH-Wert des Lösungsmittelgemischs (II) liegt erfindungsgemäß vorzugsweise in einem Bereich von 2,0 bis 8,0, insbesondere im Bereich von 2,5 bis 8,0, weiter bevorzugt im Bereich von 3,5 und 8,0, besonders bevorzugt im Bereich 5,0 bis 7,5 und ganz besonders bevorzugt im Bereich 6,0 bis 7,0.

Daher betrifft die vorliegende Erfindung auch ein wie zuvor beschriebenes Verfahren zur Reinigung eines Gasgemischs G-0 enthaltend Distickstoffmonoxid, wobei der pH-Wert des Lösungsmittelgemischs (I) oder der pH-Wert des Lösungsmittelgemischs (II) oder der pH-Wert des Lösungsmittelgemischs (I) und der pH-Wert des Lösungsmittelgemischs (II) im Bereich von 6,0 bis 7,0 liegt.

Erfindungsgemäß kann der pH-Wert des Lösungsmittelgemischs (I) oder (II) mittels jeder dem Fachmann bekannten Methode eingestellt werden. Der pH-Wert wird iedoch zumindest durch Zugabe einer Base zu dem Lösungsmittelgemisch (I) oder (II) eingestellt .

Geeignete Maßnahmen sind beispielsweise Zugabe einer Säure oder einer Lauge oder Zufuhr von Lösungsmittel bei gleichzeitiger Ausschleusung von beladenem Lösungsmittel.

Daher betrifft die vorliegende Erfindung auch ein wie zuvor beschriebenes Verfahren zur Reinigung eines Gasgemischs G-0 enthaltend Distickstoffmonoxid und NOₓ, wobei der pH-Wert des Lösungsmittelgemischs (I) oder der pH-Wert des Lösungsmittelgemischs (II) oder der pH-Wert des Lösungsmittelgemischs (I) und der pH-Wert des Lösungsmittelgemischs (II) durch Zugabe einer Base eingestellt wird.

Prinzipiell kann als Base jede erdenkliche Verbindung eingesetzt werden, deren pH-Wert als 1 Gew.-%ige Lösung in Wasser >8,0 ist. Bevorzugt werden erfindungsgemäß starke anorganische Basen, insbesondere Hydroxide, Carbonate, Hydrogencarbonate oder Phosphate von Alkali- oder Erdalkalimetallen eingesetzt. Besonders bevorzugt werden NaOH, KOH, Na₂CO₃, NaHCO₃, Na₃PO₄, K₃PO₄ eingesetzt. Weiter bevorzugt ist der Einsatz der Basen in Form einer konzentrierten wässrigen Lösung.

Geeignete Konzentrationsbereiche sind im Rahmen der vorliegenden Erfindung beispielsweise 10 bis 60 %-ige wässrige Lösungen, bevorzugt 20 bis 55 %-ige wässrige Lösungen, besonders bevorzugt 25 bis 50 %-ige wässrige Lösungen, beispielsweise 30 %-ige wässrige Lösungen, 35 %-ige wässrige Lösungen, 40 %-ige wässrige Lösungen oder 45 %-ige wässrige Lösungen.

Erfindungsgemäß besonders bevorzugt ist der Einsatz einer wässrige NaOH-Lösung als Base.

Gemäß einer bevorzugten Ausführungsform der vorliegenden Erfindung wird als Base eine 25 bis 50 %-ige wässrige NaOH-Lösung eingesetzt.

Daher betrifft die vorliegende Erfindung auch ein wie zuvor beschriebenes Verfahren zur Reinigung eines Gasgemischs G-0 enthaltend Distickstoffmonoxid und NOₓ, wobei der pH-Wert des Lösungsmittelgemischs (I) oder der pH-Wert des Lösungsmittelgemischs (II) oder der pH-Wert des Lösungsmittelgemischs (I) und der pH-Wert des Lösungsmittelgemischs (II) durch Zugabe einer Base ausgewählt aus der Gruppe bestehend aus Alkalimetallhydroxiden, Alkalimetallcarbonaten, Alkalimetallhydrogencarbonaten, Alkalimetallphosphaten, Erdalkalimetallhydroxiden, Erdalkalimetallcarbonaten, Erdalkalimetallhydrogencarbonaten und Erdalkalimetallphosphaten eingestellt wird.

Das durch das erfindungsgemäße Reinigungsverfahren erhaltene Gasgemisch G-2 enthält mindestens 50 Vol.-% N₂O, besonders bevorzugt mindestens 60 Vol.-% N₂O und ganz besonders bevorzugt mindestens 75 Vol.-% N₂O. Üblicherweise enthält das Gasgemisch G-2 bis zu 99 Vol.-% N₂O, insbesondere bis zu 97 Vol.-% N₂O, beispielsweise bis zu 96 Vol.-% N₂O, bis zu 95 Vol.-% N₂O, bis zu 94 Vol.-% N₂O, bis zu 93 Vol.-% N₂O, bis zu 92 Vol.-% N₂O, bis zu 91 Vol.-% N₂O, bis zu 90 Vol.-% N₂O oder auch bis zu 85 Vol.-% N₂O.

Im Rahmen der vorliegenden Erfindung hat das Gasgemisch G-2 beispielsweise einen Gehalt an N₂O von 60 bis 95 Vol.-%, vorzugsweise von 70 bis 90 Vol.-%, insbesondere von 75 bis 85 Vol.-%, besonders bevorzugt beispielsweise 76 Vol.-%, 77 Vol.-%, 78 Vol.-%, 79 Vol.-%, 80 Vol.-%, 81 Vol.-%, 82 Vol.-%, 83 Vol.-%, 84 Vol.-% oder 85 Vol.-%.

Das Gasgemisch G-2 hat beispielsweise einen Gehalt an CO₂ von 1 bis 20 Vol.-%, vorzugsweise von 5 bis 15 Vol.-%, besonders bevorzugt beispielsweise 6 Vol.-%, 7 Vol.-%, 8 Vol.-%, 9 Vol.-%, 10 Vol.-%, 11 Vol.-%, 12 Vol.-%, 13 Vol.-% oder 14 Vol.-%. Gleichzeitig hat das Gasgemisch G-2 beispielsweise einen Gehalt an O₂ von 0,01 bis 5,0 Vol.-%, vorzugsweise von 0,1 bis 2,5 Vol.-%, besonders bevorzugt beispielsweise 0,2 bis 1,0 Vol.-%. Darüber hinaus kann das Gasgemisch G-2 noch 0,1 bis 10 Vol.-% N₂ enthalten, vorzugsweise 0,5 bis 5 Vol.-%, sowie weitere Komponenten, beispielsweise Stickoxide oder Lösungsmittelreste. Gleichzeitig enthält das Gasgemisch G-2 weniger als 1 Vol.-% O₂, insbesondere weniger als 0,5 Vol.-% O₂, weniger als 0,5 Vol.-% NOₓ. NOₓ kann dabei beispielsweise in einer Menge von 0 bis 0,1 Vol.-% enthalten sein, vorzugsweise 0,0001 bis 0,01 Vol.-%, besonders bevorzugt 0,0002 bis 0,02 Vol.-%. Dabei ergibt die Summe der Komponenten des Gasgemischs G-2 100 Vol.-%.

Gemäß einer bevorzugten Ausführungsform des erfindungsgemäßen Verfahrens können sowohl das Lösungsmittelgemisch (I') als auch das Lösungsmittelgemisch (II') zumindest teilweise in das erfindungsgemäße Verfahren zurückgeführt werden.

Dabei ist es erfindungsgemäß möglich, dass das Lösungsmittelgemisch (I') und/oder das Lösungsmittelgemisch (II') zumindest teilweise als Lösungsmittelgemisch (I) oder (II) in das erfindungsgemäße Verfahren zurückgeführt werden. Erfindungsgemäß kann das Lösungsmittelgemisch (I') und/oder das Lösungsmittelgemisch (II') insbesondere behandelt werden, bevor es als Lösungsmittelgemisch (I) oder (II) wieder in das Verfahren eingesetzt wird. Dabei ist es insbesondere möglich, dass zunächst der pH-Wert des Lösungsmittelgemischs (I') und/oder (II') bestimmt wird und dann gegebenenfalls durch geeignete Maßnahmen der pH-Wert in dem Bereich des pH-Werts des Lösungsmittelgemischs (I) bzw. (II) eingestellt wird.

Insbesondere ist es erfindungsgemäß auch möglich, dass nur ein Teil des Lösungsmittelgemischs (I') und/oder (II') wieder in das Verfahren eingesetzt wird und beispielsweise mit Wasser oder einem anderen Lösungsmittel versetzt wird, um dann als Lösungsmittelgemisch (I) und/oder (II) wieder in das Verfahren eingesetzt zu werden.

Sofern das Lösungsmittelgemisch (I') und/oder (II') vollständig oder teilweise wieder in das Verfahren zurückgeführt wird, erfolgt eine Messung des pH-Werts vorzugsweise vor, nach oder während der Desorption gemäß Schritt A2 und/oder B2, insbesondere vor der Desorption. Die Messung des pH-Werts kann insbesondere kontinuierlich im Desorber erfolgen. Erfindungsgemäß ist es jedoch auch möglich, den pH-Wert des Lösungsmittelgemischs im Desorber zu messen.

Das Inkontaktbringen gemäß Schritt A1 bzw. B1 des erfindungsgemäßen Verfahrens und damit die Absorption kann grundsätzlich nach allen dem Fachmann bekannten Verfahren erfolgen. Insbesondere kann die Absorption im Lösungsmittelgemisch durch Erhöhung des Drucks des Eduktgases oder durch Absenkung der Temperatur des Lösungsmittelgemischs oder durch eine Kombination der genannten Maßnahmen herbeigeführt werden.

Vorzugsweise wird bei Schritt A1 bzw. B1 des erfindungsgemäßen Verfahrens zunächst das Gasgemisch komprimiert, beispielsweise auf einen Druck von 10 bis 35 bar, bevorzugt von 13 bis 30 bar, vorzugsweise von 14 bis 25 bar. Anschließend wird das komprimierte Gasgemisch vorzugsweise bei diesem Druck mit dem Lösungsmittelgemisch (I) gemäß Schritt A1 oder in dem Lösungsmittelgemisch (II) gemäß Schritt B1 in Kontakt gebracht.

Daher betrifft die vorliegende Erfindung auch ein wie zuvor beschriebenes Verfahren zur Reinigung eines Gasgemischs G-0 enthaltend Distickstoffmonoxid und NOₓ, wobei der Druck bei dem Inkontaktbringen gemäß Schritt A1 oder B1 oder A1 und B1 in einem Bereich von 10 bis 35 bar liegt.

Das Inkontaktbringen gemäß Schritt A1 und Schritt B1 erfolgt erfindungsgemäß in Einrichtungen (Absorber), in denen eine Gas-Flüssig-Phasengrenzfläche erzeugt wird, über die ein Stoff- und Wärmeübergang zwischen den Phasen ermöglicht wird, und die bei Bedarf mit internen oder externen Einrichtungen zur Wärmezufuhr und/oder Wärmeabfuhr versehen sind.

Die Führung der Phasen im Absorber kann im Gleichstrom, im Gegenstrom oder einer Kombination der genannten erfolgen.

Das Inkontaktbringen bzw. die Absorption kann erfindungsgemäß ein- oder mehrstufig durchgeführt werden, vorzugsweise einstufig. Dabei wird bei dem Inkontaktbringen vorzugsweise als Absorber eine Einrichtung mit mehreren theoretischen Trennstufen verwendet, insbesondere 2 bis 8 theoretischen Trennstufen, besonders bevorzugt 3 bis 6.

Mögliche Ausführungsformen des Absorbers sind jeweils Kolonnen mit Böden, beispielsweise Glockenböden oder Siebböden, Kolonnen mit strukturierten Einbauten, wie beispielsweise Packungen, Kolonnen mit unstrukturierten Einbauten, wie beispielsweise Füllkörpern, oder Apparate in denen die Flüssigphase dispergiert vorliegt, beispielsweise durch Versprühen in Düsen, oder eine Kombination der genannten.

Die Desorption des Gasgemischs G-1 bzw. G-2 aus der Zusammensetzung (A) bzw. Zusammensetzung (B) gemäß Schritt A2 oder B2 des erfindungsgemäßen Verfahrens kann durch Druckabsenkung über dem Lösungsmittelgemisch, Temperaturerhöhung des Lösungsmittelgemischs oder durch Strippung mit Lösungsmitteldampf oder einer Kombination der genannten herbeigeführt werden.

Die Anforderungen an die Einrichtungen (Desorber) zur Desorption des Gasgemischs G-1 bzw. G-2 aus der Zusammensetzung (A) bzw. Zusammensetzung (B), sowie die Führung der Phasen sind analog zu denen des Absorbers, d.h. geeignet sind Einrichtungen, in denen eine Gas-Flüssig-Phasengrenzfläche erzeugt wird, über die ein Stoff-und Wärmeübergang zwischen den Phasen ermöglicht wird, und die bei Bedarf mit internen oder externen Einrichtungen zur Wärmezufuhr und/oder Wärmeabfuhr versehen sind.

Die Desorption kann erfindungsgemäß ein- oder mehrstufig durchgeführt werden.

Mögliche Ausführungsformen des Desorbers sind ein einfacher (Entspannungs-)Behälter und Kolonnen.

Eine bevorzugte Ausführungsform der vorliegenden Erfindung, in der das Inkontaktbringen mit dem Lösungsmittelgemisch und die Desorption apparativ vereint sind, ist beispielsweise die Trennwandkolonne. Dabei wird das Inkontaktbringen, und damit verbunden die Absorption, und die Desorption durch Temperaturwechsel mehrstufig im Gegenstrom betrieben kombiniert mit einer Strippung mit Lösungsmitteldampf. Dabei kann sowohl gemäß A1 und A2 als auch gemäß B1 und B2 eine apparative Vereinigung des Inkontaktbringens und der Desorption erfolgen, insbesondere in einer Trennwandkolonne.

In einer bevorzugten Ausführungsform betrifft die vorliegenden Erfindung daher ein Verfahren wie oben beschrieben, wobei die Schritte A1 und A2 oder die Schritte B1 und B2 oder die Schritte A1 und A2 und die Schritte B1 und B2 in einer Trennwandkolonne durchgeführt werden.

Im Sinne einer besonders bevorzugten Ausführungsform der Erfindung wird gemäß Schritt A1 zunächst das Gasgemisch G-0 enthaltend N₂O und NOₓ unter erhöhtem Druck p_{Abso} in einer im Gegenstrom betriebenen Absorptionskolonne mit Füllkörperschüttung mit dem Lösungsmittelgemisch (I) in Kontakt gebracht, wobei eine Absorption stattfinden kann, und eine Zusammensetzung (A) erhalten wird. Gemäß Schritt A2 wird die Zusammensetzung (A) gemäß dieser Ausführungsform in einen Behälter überführt, in dem die Zusammensetzung (A) auf einen niedrigeren Druck p_{Deso} < p_{Abso} entspannt wird. Der Prozess wird vorzugsweise nahezu isotherm betrieben mit einer Temperaturdifferenz zwischen Absorptions- und Desorptionstemperatur von maximal 20 K, vorzugsweise maximal 15K, insbesondere maximal 10 K. Der Absorptionsdruck beträgt hierbei 1 bis 100 bar, bevorzugt 5 bis 65 bar, insbesondere 10 bis 40 bar, bevorzugt 10 bis 35 bar, besonders bevorzugt 13 bis 30 bar, weiter bevorzugt etwa 14 bis 25 bar und der Desorptionsdruck 0,1 bis 2 bar absolut, vorzugsweise 0,5 bis 1,5 bar absolut, besonders bevorzugt 1,0 bis 1,2 bar absolut.

Vorzugsweise wird ebenfalls gemäß Schritt B1 zunächst das Gasgemisch G-1 unter erhöhtem Druck p_{Abso} in einer im Gegenstrom betriebenen Absorptionskolonne mit Füllkörperschüttung mit einem Lösungsmittelgemisch (II) in Kontakt gebracht, wobei die Zusammensetzung (B) erhalten wird. Zusammensetzung (B) wird gemäß Schritt B2 in einen Behälter überführt, in dem die Zusammensetzung (B) auf einen niedrigeren Druck p_{Deso} < p_{Abso} entspannt wird. Der Prozess wird vorzugsweise ebenfalls nahezu isotherm betrieben mit einer Temperaturdifferenz zwischen Absorptions- und Desorptionstemperatur von maximal 20 K, vorzugsweise maximal 15 K, insbesondere maximal 10 K. Der Absorptionsdruck beträgt hierbei 1 bis 100 bar, bevorzugt 5 bis 65 bar, insbesondere 10 bis 40 bar, bevorzugt 10 bis 35 bar, besonders bevorzugt 13 bis 30 bar, weiter bevorzugt etwa 14 bis 25 bar und der Desorptionsdruck 0,1 bis 2 bar absolut, vorzugsweise 0,5 bis 1,5 bar absolut, besonders bevorzugt 1,0 bis 1,2 bar absolut.

Das Gasgemisch G-0 weist gemäß dieser Ausführungsform vorzugsweise bei dem Inkontaktbringen gemäß A1 vorzugsweise einen N₂O Gehalt von 4 bis 25 Vol.-%, vorzugsweise von 6 bis 20 Vol.-%, insbesondere von 8 bis 18 Vol.-%, besonders bevorzugt beispielsweise 9 Vol.-%, 10 Vol.-%, 11 Vo!.-%. 12 Vol.-%, 13 Vol.-%, 14 Vol.-%, 15 Vol.-%, 16 Vol.-% oder 17 Vol-% auf.

Das Gasgemisch G-0 hat beispielsweise einen Gehalt an CO₂ von 0,1 bis 7,5 Vol.-%, vorzugsweise von 0,5 bis 5 Vol.-%, besonders bevorzugt 1 bis 2,5 Vol-%. Gleichzeitig hat das Gasgemisch G-0 beispielsweise einen Gehalt an O₂ von 1 bis 10 Vol.-%, vorzugsweise von 2 bis 7,5 Vol.-%, besonders bevorzugt beispielsweise 3,0 bis 6 Vol.-%. Darüber hinaus kann das Gasgemisch G-0 noch 50 bis 95 Vol.-% N₂ enthalten, vorzugsweise 60 bis 90 Vol.-%, besonders bevorzugt 70 bis 85 Vol.-%, sowie weitere Komponenten, beispielsweise Stickoxide oder Lösungsmittelreste. NOₓ kann dabei beispielsweise in einer Menge von 0,0001 bis 0,15 Vol.-% enthalten sein, vorzugsweise 0,0005 bis 0,1 Vol.-%. Dabei ergibt die Summe der Komponenten des Gasgemischs G-0 100 Vol.-%.

Erfindungsgemäß ist es wie zuvor beschrieben möglich, dass das Lösungsmittelgemisch (I') zumindest teilweise als Lösungsmittelgemisch (I) in das Verfahren zurückgeführt wird oder dass das Lösungsmittelgemisch (II') zumindest teilweise als Lösungsmittelgemisch (II) in das Verfahren zurückgeführt wird. Dabei kann das Lösungsmittelgemisch (I') bzw. (II') vollständig oder teilweise, bevorzugt teilweise als Lösungsmittelgemisch (I) oder (II) wieder eingesetzt werden.

Erfindungsgemäß ist es insbesondere bevorzugt, dass ein Teil des Lösungsmittelgemischs (I') bzw. (II') aus dem Verfahren ausgeschleust wird. Erfindungsgemäß können beispielsweise 0,01 bis 5 % des gesamten Lösungsmittelgemischs ausgeschleust werden, bevorzugt 0,05 bis 2,5 %, insbesondere 0,1 bis 1,0 %, beispielsweise 0,2 %, 0,3 %, 0,4 %, 0,5 %, 0,6 %, 0,7 %, 0,8 % oder 0,9 %.

Erfindungsgemäß ist es bevorzugt, dass das Lösungsmittelgemisch (I') bzw. (II') teilweise zurückgeführt wird, wobei jeweils andere Verbindungen, insbesondere Wasser, zu dem Lösungsmittelgemisch zugesetzt werden können. Dabei werden andere Verbindungen in einer Menge zugesetzt, die der Menge des Lösungsmittelgemischs (I') bzw. (II') entspricht, die nicht in das Verfahren zurückgeführt wird. Der pH-Wert wird dann erfindungsgemäß auf einen für das Lösungsmittelgemisch (I) bzw. (II) geeigneten Bereich eingestellt. Erfindungsgemäß ist es möglich, dass die Messung des pH-Werts kontinuierlich erfolgt.

Erfindungsgemäß ist es auch möglich, das Lösungsmittelgemisch (I') bzw. (II') vollständig als Lösungsmittelgemisch (I) oder (II) wieder einzusetzen, sofern gewährleistet ist, dass der pH-Wert des Lösungsmittelgemischs (I) bzw. (II) in dem erfindungsgemäßen Bereich eingestellt wird. Dabei ist es erfindungsgemäß insbesondere möglich, dass durch geeignete Maßnahmen einzelne Verbindungen aus dem Lösungsmittelgemisch (I') oder (II') entfernt werden, beispielsweise Salze oder Lösungsmittel, bevor das jeweilige Gemisch als Lösungsmittelgemisch (I) oder (II) in das Verfahren zurückgeführt wird.

Daher betrifft die vorliegende Erfindung auch ein wie zuvor beschriebenes Verfahren zur Reinigung eines Gasgemischs G-0 enthaltend Distickstoffmonoxid und NOₓ, wobei das Lösungsmittelgemisch (I') zumindest teilweise als Lösungsmittelgemisch (I) in das Verfahren zurückgeführt wird, oder wobei das Lösungsmittelgemisch (II') zumindest teilweise als Lösungsmittelgemisch (II) in das Verfahren zurückgeführt wird, oder wobei das Lösungsmittelgemisch (I') zumindest teilweise als Lösungsmittelgemisch (I) in das Verfahren zurückgeführt wird und das Lösungsmittelgemisch (II') zumindest teilweise als Lösungsmittelgemisch (II) in das Verfahren zurückgeführt wird.

Bei dieser Verfahrensführung wird das erfindungsgemäße Verfahren bevorzugt als kontinuierliches Verfahren ausgeübt.

In einer besonders bevorzugten kontinuierlichen Ausführung des Verfahrens wird ein Teil des Lösungsmittelgemischs (I') bzw. (II') ausgeschleust und durch entsalztes Wasser ersetzt. Die Menge des Lösungsmittelgemischs, die auszuschleusen ist, und die Menge an frischem Lösungsmittel, insbesondere an Wasser, das zuzufügen ist, werden so bemessen, dass einerseits die Menge an Lösungsmittelgemisch (I) bzw. (II) im System annährend konstant bleibt und andererseits die Menge an gelösten Salzen im Lösungsmittelgemisch (I) bzw. im Lösungsmittelgemisch (II) vorzugsweise nicht über 10 Gew.-% ansteigt und besonders bevorzugt nicht über 7 Gew.-%, jeweils bezogen auf das gesamte Lösungsmittelgemisch (I) bzw. (II), ansteigt.

Neben den Schritten A1, A2, B1 und B2 kann das erfindungsgemäße Verfahren auch weitere Schritte umfassen. So kann das Verfahren beispielsweise auch eine weitere Behandlung des Gasgemischs G-1 zwischen den Schritten A2 und B1 umfassen. Derartige Behandlungen umfassen beispielsweise eine Änderung der Temperatur oder eine Änderung des Drucks oder eine Änderung der Temperatur und des Drucks.

Dabei kann sich beispielsweise die Zusammensetzung eines Gasgemischs ändern, beispielsweise durch Kondensation einer der Komponenten. Bei diesen Komponenten kann es sich beispielsweise um Wasser oder eine andere im Lösungsmittelgemisch (I) enthaltene Verbindung handeln, vorzugsweise um ein Lösungsmittel, das im Rahmen des erfindungsgemäßen Verfahrens im Lösungsmittelgemisch (I) für das Inkontaktbringen gemäß Schritt A1 eingesetzt wird.

Erfindungsgemäß ist es möglich, dass aus dem Gasgemisch G-1 oder G-2 weitere Komponenten abgetrennt werden. So ist es beispielsweise möglich, dass aus dem Gasgemisch G-2 durch Kompression und anschließendes Abkühlen Spuren von Wasser abgetrennt werden, die nach der Desorption gemäß Schritt B2 im Gasgemisch G-2 enthalten sein können.

Dabei wird das Gasgemisch G-2 vorteilhafterweise auf einen Druck von 1 bis 35 bar komprimiert, bevorzugt 2 bis 30 bar, weiter bevorzugt 3 bis 27 bar. Eine Abkühlung erfolgt vorzugsweise anschließend, bevorzugt auf 1 bis 25 °C, besonders bevorzugt 3 bis 20 °C, insbesondere 4 bis 15°C, weiter bevorzugt 8 bis 12°C.

Ebenso ist es im Rahmen der vorliegenden Erfindung möglich, im Anschluss an die Schritte A1, A2, B1 und B2 beispielsweise eine Teilkondensation von Distickstoffmonoxid durchzuführen oder eine Rektifikation, insbesondere zur Abtrennung von im Gasgemisch G-2 verbleibenden Leichtsiedern wie beispielsweise Sauerstoff und Stickstoff.

Erfindungsgemäß kann das durch das erfindungsgemäße Verfahren gereinigte Gasgemisch G-2 enthaltend Distickstoffmonoxid in einer weiteren Umsetzung eingesetzt werden. Dazu kann das Gasgemisch gasförmig eingesetzt werden. Es ist jedoch auch möglich, das erhaltene Gasgemisch zunächst derart zu behandeln, dass das Gasgemisch in flüssiger oder überkritischer Form vorliegt, und dann in einer weiteren Umsetzung eingesetzt wird. Das Gasgemisch kann durch geeignete Wahl des Drucks oder der Temperatur verflüssigt werden.

Damit betrifft die vorliegende Erfindung auch ein Verfahren, wobei das erhaltene Gasgemisch G-2 verflüssigt wird.

Vorzugsweise wird dazu das Gasgemisch G-2 zunächst komprimiert und anschließend gekühlt. Dabei wird das Gasgemisch G-2 vorteilhafterweise auf einen Druck von 1 bis 35 bar komprimiert, bevorzugt 2 bis 30 bar, weiter bevorzugt 3 bis 27 bar. Eine Abkühlung erfolgt vorzugsweise anschließend, bevorzugt auf 10 bis -70 °C, besonders bevorzugt 8 bis -30 °C, insbesondere 5 bis -25°C.

Das erfindungsgemäße Verfahren hat unter anderem den Vorteil, dass CO₂ neben der im Vergleich zu anderen Inertgasen guten Inertisierungswirkung eine dem N₂O sehr ähnliche Siedekurve und ähnliche kritische Parameter aufweist. Dadurch lässt sich das im erfindungsgemäßen Verfahren erhaltene Gasgemisch G-2 enthaltend N₂O und gegebenenfalls CO₂ bei einer höheren Temperatur kondensieren, als ein vergleichbares Gemisch aus N₂O und einem anderen Inertgas. Durch die sehr ähnlichen Siedekurven weist das kondensierte Gasgemisch fast das gleiche Verhältnis von N₂O zu CO₂ auf wie das gasförmige Gemisch, so dass das Inertisierungsmittel in der flüssigen Phase erhalten bleibt.

Darüber hinaus weist CO₂ eine gute Löslichkeit in organischen Verbindungen auf, so dass ein geringerer Druck ausreicht, um bei einer Reaktion in flüssiger Phase die Bildung einer Gasphase im Reaktor zu vermeiden.

Das nach einem erfindungsgemäßen Verfahren erhaltene Gasgemisch G-2 enthaltend Distickstoffmonoxid kann grundsätzlich für alle Anwendungen eingesetzt werden, bei denen üblicherweise reine Distickstoffmonoxidströme oder mit Inertgas versetzte Distickstoffmonoxidströme eingesetzt werden. Insbesondere eignen sich die Gasgemische beispielsweise für die Oxidation von Methanol zu Formaldehyd wie beispielsweise in der EP-A 0 624 565 oder DE-A 196 05 211 beschrieben. Daher betrifft die vorliegende Erfindung auch die Verwendung der nach einem erfindungsgemäßen Verfahren erhältlichen Gasgemische enthaltend Distickstoffmonoxid als Oxidationsmittel für Methanol.

Durch das erfindungsgemäße Verfahren werden Gasgemische G-2 enthaltend Distickstoffmonoxid erhalten, die einen besonders geringen Anteil an störenden Nebenkomponenten enthalten. Dies ist insbesondere für die Verwendung des Gasgemischs enthaltend Distickstoffmonoxid als Oxidationsmittel vorteilhaft, da durch den geringen Anteil an störenden Nebenkomponenten kaum Nebenreaktionen auftreten und somit bei einer Oxidation besonders reine Produkte erhalten werden können. Vorzugsweise enthält das Gasgemisch G-2 nach der erfindungsgemäßen Reinigung neben Distickstoffmonoxid auch Kohlenstoffdioxid in geeigneten Mengen.

Das erfindungsgemäß gereinigte Gasgemisch G-2 enthält vorzugsweise 50 bis 99,0 Vol.-% Distickstoffmonoxid, 1 bis 20 Vol.-% Kohlenstoffdioxid und 0 bis 25 Vol.-% weitere Gase. Die angegebenen Vol.-% beziehen sich jeweils auf das gesamte Gasgemisch G-2. Die Summe der einzelnen Komponenten des Gasgemischs G-2 ergibt dabei 100 Vol.-%.

Vorzugsweise enthält das erfindungsgemäß gereinigte Gasgemisch G-2 60 bis 95 Vol.-% Distickstoffmonoxid, insbesondere 70 bis 90 Vol.-%, besonders bevorzugt 75 bis 89 Vol.-% Distickstoffmonoxid.

Das erfindungsgemäß gereinigte Gasgemisch G-2 enthält weiterhin 1 bis 20 Vol.-% Kohlenstoffdioxid. Vorzugsweise enthält das Gasgemisch G-2 5 bis 15 Vol.-% Kohlenstoffdioxid, insbesondere 6 bis 14 Vol.-% Kohlenstoffdioxid.

Vorzugsweise enthält das Gasgemisch G-2 0 bis 25 Vol.-% weitere Gase. Das erfindungsgemäß gereinigte Gasgemisch G-2 kann ein oder mehrere weitere Gase enthalten, wobei die angegebene Menge auf die Summe der enthaltenen Gase bezogen ist. Das Gasgemisch G-2 kann beispielsweise Sauerstoff, Stickstoff und Wasser enthalten.

Es wurde gefunden, dass bei der Anwesenheit von CO₂ als Inertgas in Gasgemischen enthaltend N₂O im Vergleich zu anderen Inertgasen deutlich geringere Mengen des Inertgases, also Kohlenstoffdioxid, benötigt werden, um einen sicheren Betrieb zu gewährleisten, beispielsweise um einen Selbstzerfall von Distickstoffmonoxid zu unterbinden.

Daher betrifft die vorliegende Erfindung auch die Verwendung eines Gasgemisches das nach einem erfindungsgemäßen Verfahren wie oben beschrieben erhalten wird als Oxidationsmittel, insbesondere als Oxidationsmittel für Olefine.

Insbesondere betrifft die vorliegende Erfindung auch die Verwendung eines Gasgemischs enthaltend 50 bis 99,0 Vol.-% Distickstoffmonoxid, 1 bis 20 Vol.-% Kohlenstoffdioxid und 0 bis 25 Vol.-% weitere Gase als Oxidationsmittel, insbesondere als Oxidationsmittel für Olefine, wobei derartige Gasgemische durch das erfindungsgemäße Reinigungsverfahren erhalten werden.

Grundsätzlich eignen sich die erfindungsgemäß erhaltenen Gasgemische enthaltend Distickstoffmonoxid zur Oxidation von Olefinen. Geeignete Olefine sind beispielsweise offenkettige oder cyclische Olefine mit einer oder mehreren Doppelbindungen. Weiter bevorzugt sind cyclische Olefine mit einer oder mehreren Doppelbindungen, beispielsweise Cyclopenten, Cyclohexen, Cyclohepten, Cycloocten, Cyclodecen, Cycloundecen, Cyclododecen, 1,4-Cyclohexadien, 1,5-Cyclooctadien, 1,5-Cyclododecadien oder 1,5,9-Cyclododecatrien.

Daher betrifft die vorliegende Erfindung gemäß einer bevorzugten Ausführungsform auch eine wie zuvor beschriebene Verwendung als Oxidationsmittel für Olefine, wobei das Olefin ausgewählt ist aus der Gruppe bestehend aus Cyclopenten, Cyclododecen und 1,5,9-Cyclododecatrien.

Dieser angereicherte und gereinigte N₂O-haltige Gasstrom G-2 eignet sich ganz besonders zur Oxidation von Olefinen zu Ketonen. Für diesen Zweck kann vorzugsweise entweder das Gasgemisch G-2 direkt in das zu oxidierende Olefin oder ein anderes Lösungsmittel absorbiert werden oder das Gasgemisch G-2 kann zuerst verflüssigt werden, bevor es mit dem Olefin zur Reaktion gebracht wird.

Insbesondere beim Einsatz eines verflüssigten Gasgemischs G-2 ist es vorteilhaft, wenn der Anteil an Inertgasen im Gasgemisch G-2 möglichst gering ist, da ansonsten das Reaktorvolumen unnötig vergrößert wird.

Für die erfindungsgemäße Verwendung als Oxidationsmittel, insbesondere für Olefine, kann die Oxidation generell gemäß sämtlicher Verfahrensführungen erfolgen, bei denen die Oxidation, insbesondere des Olefins, stattfindet. Insbesondere sind sowohl kontinuierliche Verfahrensführungen und Fahrweisen der Umsetzung als auch Batch-Reaktion möglich. Die Reaktionsbedingungen für die Oxidation werden erfindungsgemäß so gewählt, dass eine Reaktion stattfindet. Druck und Temperatur können entsprechend gewählt werden.

Vorzugsweise liegt der Druck in einem Bereich bis 350 bar, beispielsweise 1 bis 320 bar, bevorzugt 2 bis 300 bar, insbesondere 3 bis 280 bar. Die Temperatur liegt vorzugsweise in einem Bereich von 220 bis 320°C, beispielsweise 230 bis 300°C, insbesondere 240 bis 290°C.

Dabei kann die Oxidation in Gegenwart eines geeigneten Lösungsmittels durchgeführt werden. Es ist erfindungsgemäß aber ebenso möglich, die Oxidation ohne den Zusatz eines Lösungsmittels durchzuführen.

Es ist jedoch im Rahmen der vorliegenden Erfindung auch möglich, dass das Gasgemisch G-2 durch geeignete Wahl des Drucks und/oder der Temperatur verflüssigt wird oder in einen überkritischen Zustand gebracht wird. Das verflüssigte Gasgemisch kann dann direkt in die Oxidation eingesetzt werden.

Vorzugsweise wird die Oxidation erfindungsgemäß in diesem Fall durch geeignete Wahl des Drucks und der Temperatur so geführt, dass in der Reaktionszone keine Gasphase auftritt.

Vorzugsweise liegt der Druck in einem Bereich bis 350 bar, beispielsweise 1 bis 320 bar, bevorzugt 2 bis 300 bar, insbesondere 3 bis 280 bar. Die Temperatur liegt vorzugsweise in einem Bereich von 220 bis 320°C, beispielsweise 230 bis 300°C, insbesondere 240 bis 290°C.

Daher betrifft die vorliegende Erfindung auch ein Verfahren zur Herstellung eines Ketons, mindestens umfassend die folgenden Schritte
A1 Inkontaktbringen eines Gasgemisches G-0 enthaltend Distickstoffmonoxid und NOₓ mit einem Lösungsmittelgemisch (I) mindestens enthaltend 50 Gew.-% Wasser bezogen auf das gesamte Lösungsmittelgemisch (I), wobei der pH-Wert des Lösungsmittelgemischs (I) im Bereich von 3,5 bis 8,0 liegt, unter Erhalt einer Zusammensetzung (A);
A2 Desorption eines Gasgemisches G-1 aus der Zusammensetzung (A) unter Erhalt eines Lösungsmittelgemischs (I');
B1 Inkontaktbringen des Gasgemisches G-1 mit einem Lösungsmittelgemisch (II) mindestens enthaltend 50 Gew.-% Wasser bezogen auf das gesamte Lösungsmittelgemisch (II), wobei der pH-Wert des Lösungsmittelgemischs (II) im Bereich von 2,0 bis 8,0 liegt, unter Erhalt einer Zusammensetzung (B);
B2 Desorption eines Gasgemisches G-2 aus der Zusammensetzung (B) unter Erhalt eines Lösungsmittelgemischs (II');
C Inkontaktbringen des Gasgemisches G-2 mit mindestens einem Olefin;
wobei sich der pH-Wert jeweils auf eine Messung mit einer Glaselektrode bezieht,
wobei der pH-Wert des Lösungsmittelgemischs (I) oder der pH-Wert des Lösungsmittelgemischs (II) oder der pH-Wert des Lösungsmittelgemischs (I) und der pH-Wert des Lösungsmittelgemischs (II) durch Zugabe einer Base eingestellt wird.

Für die bevorzugten Ausführungsformen der Schritte A1, A2 und B1 und B2 gelten die vorstehenden Ausführungen.

Ebenso ist es im Rahmen der vorliegenden Erfindung möglich, dass das Verfahren mehrere Schritte A1 und A2 oder mehrere Schritte B1 und B2 umfasst.

Die Umsetzung gemäß Schritt C kann generell gemäß sämtlicher Verfahrensführungen erfolgen, bei denen das Olefin und das Gasgemisch G-2 enthaltend Distickstoffmonoxid miteinander reagieren. Insbesondere sind sowohl kontinuierliche Verfahrensführungen und Fahrweisen der Umsetzung als auch Batch-Reaktion möglich. Die Reaktionsbedingungen für Schritt C werden erfindungsgemäß so gewählt, dass eine Reaktion des mindestens einen Olefins mit dem erfindungsgemäß gereinigten Gasgemisch stattfindet. Druck und Temperatur können entsprechend gewählt werden.

Dabei kann die Reaktion in Gegenwart eines geeigneten Lösungsmittels durchgeführt werden. Es ist erfindungsgemäß aber ebenso möglich, die Umsetzung gemäß Schritt C ohne den Zusatz eines Lösungsmittels durchzuführen.

Erfindungsgemäß ist es jedoch auch möglich, dass das Verfahren zur Herstellung eines Ketons weitere Schritte umfasst. So kann das Gasgemisch G-2 beispielsweise vor Schritt C und nach den Schritten A1, A2 und B1 und B2 behandelt werden. Eine mögliche Behandlung ist beispielsweise eine Änderung von Druck und/oder Temperatur des Gasgemischs. Eine weitere mögliche Behandlung ist beispielsweise die Absorption des Gasgemischs G-2 in einem Lösungsmittel, so dass das absorbierte Gasgemisch G-2 in Schritt C eingesetzt werden kann. Dabei kann das Lösungsmittel jedes geeignete Lösungsmittel sein. Bevorzugt handelt es sich bei dem Lösungsmittel um das Olefin, das gemäß Schritt C oxidiert werden soll.

Es ist jedoch im Rahmen der vorliegenden Erfindung auch möglich, dass das Gasgemisch G-2 enthaltend Distickstoffmonoxid vor Schritt C und nach den Schritten A1, A2 und B1 und B2 durch geeignete Wahl des Drucks und/oder der Temperatur verflüssigt wird oder in einen überkritischen Zustand gebracht wird. Das verflüssigte Gasgemisch enthaltend Distickstoffmonoxid kann dann gemäß Schritt C direkt mit dem Olefin in Kontakt gebracht werden.

Daher betrifft die vorliegende Erfindung gemäß einer weiteren Ausführungsform auch ein Verfahren zur Herstellung eines Ketons wie oben beschrieben, wobei das in Schritt C eingesetzte Gasgemisch verflüssigt ist.

Grundsätzlich können in Schritt C des erfindungsgemäßen Verfahrens alle geeigneten Olefine eingesetzt werden, beispielsweise Olefine mit 2 bis 18 C-Atomen, insbesondere Olefine mit 5 bis 12 C-Atomen. Geeignete Olefine sind beispielsweise offenkettige oder cyclische Olefine mit einer oder mehreren Doppelbindungen. Weiter bevorzugt sind cyclische Olefine mit einer oder mehreren Doppelbindungen, beispielsweise Cyclopenten, Cyclohexen, Cyclohepten, Cycloocten, Cyclodecen, Cycloundecen, Cyclododecen, 1,4-Cyclohexadien, 1,5-Cyclooctadien, 1,5-Cyclododecadien oder 1,5,9-Cyclododecatrien.

Besonders bevorzugt wird als Olefin Cyclopenten, Cyclododecen oder 1,5,9-Cyclododecatrien eingesetzt. Daher betrifft die vorliegende Erfindung in einer bevorzugten Ausführungsform ein Verfahren zur Herstellung eines Ketons wie oben beschrieben, wobei das Olefin ausgewählt ist aus der Gruppe bestehend aus Cyclopenten, Cyclododecen und 1,5,9-Cyclododecatrien.

Im Folgenden wird die Erfindung anhand von Beispielen näher erläutert.

### BEISPIELE

Bei den folgenden Beispielen werden die Zusammensetzungen von Gasgemischen immer abzüglich von Wasser angegeben (Trockengaszusammensetzung), sofern nicht anders angegeben. Die Gemische sind bei der angegebenen Temperatur und Druck mit Wasser gesättigt, sofern nicht anders angegeben.

Die Zusammensetzungen der Gasgemische werden als Vol.-% angegeben es sei denn es werden andere Einheiten explizit angegeben.

pH-Werte wurden mit einer kommerziellen temperaturkorrigierten Glaselektrode gemessen, die vorher gegen bekannte pH-Standards kalibriert wurde.

Im Folgenden wird unter NOₓ die Summe aus NO₂ und NO verstanden. Folgende nasschemische Methode wurde zur Bestimmung genutzt: Bei 0°C und 1023 mbar absolut wurde ein Gasprobenahmezylinder aus Glas mit 250 ml Inhalt nach Probennahme mit 30 %iger wässriger H₂O₂-Lösung und 10 %iger wässriger Salzsäure versetzt und geschüttelt. NO und NO₂ wurden dabei zu NO₃⁻ oxidiert. Der Gaszylinderinhalt wurde anschließend mit Wasser ausgespült und Nitrat ionenchromatographisch bestimmt.

Der relative Anreicherungsfaktor gamma_{O2} wird definiert als [N₂O]/[O₂] im Produktgas im Verhältnis zu [N₂O]/[O₂] im zugeführten Gasstrom. Dieser Faktor ist ein Maß für die Güte der Trennung zwischen N₂O und O₂ (Anreicherung von N₂O und Abreicherung von O₂). Je höher der Wert von gamma, desto effektiver ist die Trennung. Entsprechend können gamma-Faktoren auch für andere Komponenten definiert werden.

### Beispiel 1 (nicht erfindungsgemäß)

Als Feed für die Versuche wurde das Abgas einer Druckabsorptionskolonne zur Salpetersäure-Synthese eingesetzt, die wiederum als Feed ein Gemisch, beinhaltend das Abgas einer Adipinsäure-Fabrik, nutzte, das mit NO aufgestockt wurde.

Das Gasgemisch wurde mit Umgebungstemperatur (maximal 40°C) und 7 bar Vordruck angeliefert und war mit Wasser gesättigt. Dieses Gemisch wurde zuerst auf 5°C abgekühlt und das kondensierte Wasser (Salpetersäure) abgetrennt. Das getrocknete Gas wurde dann auf 25 bar komprimiert und auf 35°C abgekühlt. Dieser in die Absorption eingesetzte Gasstrom hatte folgende Zusammensetzung: 11,9 Vol.-% N₂O, 81,5 Vol.-% N₂, 4,3 Vol.-% O₂, 1,6 Vol.-% CO₂, 0,6 Vol.-% Ar, 0,07 Vol.-% CO, 300 vppm NOₓ.

Dieses Gemisch wurde dann in einer mit einer Packung gefüllten (Kühni Rombopak 9M) Kolonne mit einem Durchmesser von 77,9 mm und einer Höhe von 1800 mm mit Wasser in Kontakt gebracht, wobei das Wasser am Anfang des Versuchs einen pH-Wert von 8,0 aufwies der rasch auf 0,3 absank. Die Kolonne wurde bei einer Temperatur von 35°C im Gegenstrom betrieben. Das Gemisch wurde mengengeregelt (2 kg/h) von unten eingespeist und druckgeregelt von oben entnommen. Das von oben entnommene Gasgemisch war an N₂O abgereichert und wurde entsorgt. Die Zusammensetzung des entsorgten Gasgemischs nach Erreichen des stationären Zustandes war wie folgt: 6,8 Vol.-% N₂O, 87,2 Vol.-% N₂, 4,4 Vol.-% O₂, 0,8 Vol.-% CO₂, 0,7 Vol.-% Ar, 0,02 Vol.-% CO, 6 vppm NOₓ.

Von oben wurde Wasser als Absorptionsmittel mengengeregelt (200 kg/h) zugegeben. Das beladene Wasser wurde von unten standgeregelt entnommen und in einem Desorberturm entspannt. Der Desorberturm hatte ein Durchmesser von 150 mm und eine Höhe von 300 mm. Der Desorberturm wurde bei 35°C und 1 bar betrieben.

Das desorbierte Gas war angereichert in N₂O und hatte nach Erreichen des stationären Zustandes folgende Zusammensetzung: 54,9 Vol.-% N₂O, 33,1 Vol.-% N₂, 3,1 Vol.-% O₂, 8,3 Vol.-% CO₂, 0,5 Vol.-% Ar, 0,03 Vol.-% CO, 110 vppm NOₓ.

Das entladene Absorptionsmittel wurde vollständig zurückgeführt. Es wurde lediglich so viel frisches vollentsalztes Wasser, das mit < 4 ppm Ammoniak dotiert wurde und einen pH-Wert zwischen 9,2 und 9,8 aufwies, eingespeist wie nötig war, um die Wassermenge im System konstant zu halten (ca. 2 kg Wasser).

Mit fortschreitender Versuchsdauer sank der pH-Wert des als Absorptionsmittel verwendeten Wassers immer weiter ab und erreicht nach 0,5 bis 1 h einen stationären Wert von 0,3.

Das erhaltene Absorptionsmittel war korrosiv und nur mit korrosionsfesten Apparaten zu betreiben. Der relative Anreicherungsfaktor gamma_{O2} betrug 6,4.

### Beispiel 2 (nicht erfindungsgemäß)

Das Beispiel wurde wie oben angegeben durchgeführt mit dem Unterschied, dass 5 kg/h des Absorptionsmittels nach der Desorption ausgeschleust und durch frisches vollentsalztes Wasser, das mit < 4 ppm Ammoniak dotiert wurde und einen pH-Wert zwischen 9,2 und 9,8 aufwies, ersetzt wurden. Durch diese Maßnahme wurde der pH-Wert des Absorptionsmittels im stationären Zustand auf 2,7 stabil gehalten.

Der in die Absorption eingesetzte Gasstrom hatte folgende Zusammensetzung: 11,5 Vol.-% N₂O, 82,5 Vol.-% N₂, 3,6 Vol.-% O₂, 1,6 Vol.-% CO₂, 0,7 Vol.-% Ar, 0,08 Vol.-% CO, 225 vppm NOₓ.

Der nach der Desorption erhaltene Gasstrom hatte die folgende Zusammensetzung: 61,3 Vol.-% N₂O, 26,9 Vol.-% N₂, 1,9 Vol.-% O₂, 9,4 Vol.-% CO₂, 0,4 Vol.-% Ar, 0,04 Vol.-% CO, 69 vppm NOₓ.

Der relativer Anreicherungsfaktor gamma_{O2} betrug 9,9, der Verbrauch an Absorptionsmittel lag bei19 kg Wasser/kg N₂O.

### Beispiel 3

Beispiel 3 wurde analog Beispiel 1 durchgeführt. Im Unterschied zu Beispiel 1 wurde zum Absorptionsmittel nach dem Desorberturm eine 10%igen wässrige NaOH-Lösung zudosiert, um den pH-Wert konstant auf 6,0 zu halten. Es wurde ein fester Teilstrom an Absorptionsmittel von 2 kg/h ausgeschleust. Um die Menge an Absorptionsmittel im System konstant zu halten, wurde nach Bedarf frisches vollentsalztes Wasser, das mit < 4 ppm Ammoniak dotiert wurde und einen pH-Wert zwischen 9,2 und 9,8 aufwies, ergänzt.

Der in die Absorption eingesetzte Gasstrom hatte folgende Zusammensetzung: 13,2 Vol.-% N₂O, 80,5 Vol.-% N₂, 3,7 Vol.-% O₂, 1,7 Vol.-% CO₂, 0,8 Vol.-% Ar, 0,08 Vol.-% CO, 172 vppm NOₓ.

Der nach der Desorption erhaltene Gasstrom hatte die folgende Zusammensetzung: 62,3 Vol.-% N₂O, 26,0 Vol.-% N₂, 2,1 Vol.-% O₂, 8,9 Vol.-% CO₂, 0,6 Vol.-% Ar, 0,04 Vol.-% CO, 4 vppm Vol.-% NOₓ.

Der relative Anreicherungsfaktor gamma_{O2} betrug 8,2. Der Verbrauch an Absorptionsmittel lag bei 7,4 kg/kg N₂O und es wurden 0,5 g/h NaOH benötigt, um den pH-Wert im stationären Zustand konstant zu halten. Die stationäre Konzentration an gelösten Salzen im Absorptionsmittel betrug nur 0,1 Gew.-%.

### Beispiel 4 (nicht erfindungsgemäß)

Beispiel 4 wurde analog zu Beispiel 3 durchgeführt, aber der pH-Wert wurde konstant bei 9,0 gehalten. Es wurde ebenfalls ein fester Teilstrom an Absorptionsmittel von 2 kg/h ausgeschleust. Um die Menge an Absorptionsmittel im System konstant zu halten, wurde nach Bedarf frisches vollentsalztes Wasser, das mit < 4 ppm Ammoniak dotiert wurde und einen pH-Wert zwischen 9,2 und 9,8 aufwies, ergänzt.

Der in die Absorption eingesetzte Gasstrom hatte folgende Zusammensetzung: 14, 5 Vol.-% N₂O, 78,8 Vol.-% N₂, 3,7 Vol.-% O₂, 2,0 Vol.-% CO₂, 0,9 Vol.-% Ar, 0,08 Vol.-% CO, 185 vppm NOₓ.

Der nach der Desorption erhaltene Gasstrom hatte die folgende Zusammensetzung: 69,3 Vol.-% N₂O, 24,3 Vol.-% N₂, 2,0 Vol.-% O₂, 3,8 Vol.-% CO₂, 0,5 Vol.-% Ar, 0,04 Vol.-% CO, 4 vppm NOₓ.

Der relative Anreicherungsfaktor gamma_{O2} betrug 9,0. Der Verbrauch an Absorptionsmittel lag bei 7,7 kg/kg N₂O.

Der Bedarf an NaOH, um den pH-Wert konstant zu halten, lag bei 42 g/h NaOH. Die stationäre Konzentration an gelösten Salzen im Absorptionsmittel war mit 6 Gew.-% deutlich höher als im Beispiel 3. CO₂ wurde im Vergleich zu Beispiel 3 stärker abgereichert.

### Beispiel 5

Beispiel 5 wurde analog Beispiel 3 durchgeführt. Darüber hinaus wurde jedoch das nach der ersten Desorptionsstufe erhaltene Gas in einer zweiten Konzentrierungstufe, die in ihrer grundsätzlichen Funktion analog zur ersten Konzentrierungstufe aufgebaut war, weiter aufkonzentriert. Die zweite Konzentrierungsstufe war jedoch nicht mit Na-OH-Einspeisung und einer pH-Wert Regelung ausgestattet.

Der in die erste Absorption eingesetzte Gasstrom hatte folgende Zusammensetzung: 13,7 Vol.-% N₂O, 78,4 Vol.-% N₂, 5,6 Vol.-% O₂, 1,7 Vol.-% CO₂, 0,8 Vol.-% Ar, 0,08 Vol.-% CO, 380 vppm NOₓ.

Die erste Konzentrierungsstufe wurde analog Beispiel 3 betrieben. Dabei wurde nach der ersten Desorptionsstufe ein Gasstrom mit der folgenden Zusammensetzung erhalten: 59,5 Vol.-% N₂O, 24,1 Vol.-% N₂, 3,0 Vol.-% O₂, 7,5 Vol.-% CO₂, 0,5 Vol.-% Ar, 0,04 Vol.-% CO, 5 vppm NOₓ.

Dieser Gasstrom wurde auf 25 bar verdichtet, auf 35 °C abgekühlt und in den Absorber der nachgeschalteten zweiten Konzentrierungsstufe eingespeist. Der Absorber der zweiten Stufe war, wie auch der der ersten Stufe, als Kolonne, mit Durchmesser von 50 mm ausgeführt und mit einer Packung (Kühni Rombopak 9M) bestückt mit einer Höhe von 1200 mm. Im Absorber wurde das Gas mit Wasser (ca. 164 g(H2O)/g(eingespeistes N₂O)) in Kontakt gebracht. Die Kolonne wurde bei einer Temperatur von 35°C und einem Druck von 25 bar im Gegenstrom betrieben. Das dem zweiten Absorber oben entnommene Gasgemisch wurde entsorgt.

Der pH-Wert des Wassers in der zweiten Absorptionsstufe blieb während des Versuchs bei 7,0 ± 0,5.

Das beladene Wasser wurde in einen zweiten Desorber auf einen Druck von etwa 1,10 bar entspannt. Dabei wurdeein Produktgasstrom mit folgender Zusammensetzung erhalten: 81,7 Vol.-% N₂O, 1,7 Vol.-% N₂, 0,45 Vol.-% O₂, 10,7 Vol.-% CO₂, 0,1 Vol.-% Ar, 0,005 Vol.-% CO.

Das entladene Absorptionsmittel wurde vollständig zurückgeführt. Es wurde lediglich so viel frisches vollentsalztes Wasser, das mit < 4 ppm Ammoniak dotiert wurde und einen pH-Wert zwischen 9,2 und 9,8 aufwies, eingespeist wie nötig war, um die Wassermenge im System konstant (ca. 20 g/h) zu halten.

Bezogen auf die in die erste Konzentrierungsstufe eingespeiste N₂O-Menge wurde mit dieser Versuchsanordnung eine N₂O-Ausbeute von 65 % erzielt.

## Patentansprüche

1. Verfahren zur Reinigung eines Gasgemisches G-0 enthaltend Distickstoffmonoxid und NOₓ, mindestens umfassend die folgenden Schritte:
A1 Inkontaktbringen des Gasgemisches G-0 mit einem Lösungsmittelgemisch (I) mindestens enthaltend 50 Gew.-% Wasser bezogen auf das gesamte Lösungsmittelgemisch (I), wobei der pH-Wert des Lösungsmittelgemischs (I) im Bereich von 3,5 bis 8,0 liegt, unter Erhalt einer Zusammensetzung (A);
A2 Desorption eines Gasgemisches G-1 aus der Zusammensetzung (A) unter Erhalt eines Lösungsmittelgemischs (I');
B1 Inkontaktbringen des Gasgemisches G-1 mit einem Lösungsmittelgemisch (II) mindestens enthaltend 50 Gew.-% Wasser bezogen auf das gesamte Lösungsmittelgemisch (II), wobei der pH-Wert des Lösungsmittelgemischs (II) im Bereich von 2,0 bis 8,0 liegt, unter Erhalt einer Zusammensetzung (B);
B2 Desorption eines Gasgemisches G-2 aus der Zusammensetzung (B) unter Erhalt eines Lösungsmittelgemischs (II');
wobei sich der pH-Wert jeweils auf eine Messung mit einer Glaselektrode bezieht,
wobei der pH-Wert des Lösungsmittelgemischs (I) oder der pH-Wert des Lösungsmittelgemischs (II) oder der pH-Wert des Lösungsmittelgemischs (I) und der pH-Wert des Lösungsmittelgemischs (II) durch Zugabe einer Base eingestellt wird.

2. Verfahren nach Anspruch 1, wobei das Gasgemisch G-0 das Abgas einer Adipinsäureanlage und/oder einer Dodecandicarbonsäureanlage und/oder einer Hydroxylaminanlage und/oder einer mit dem Abgas einer Adipinsäureanlage und/oder einer Dodecandicarbonsäureanlage und/oder einer Hydroxylaminanlage betriebenen Salpetersäureanlage ist.

3. Verfahren nach einem der Ansprüche 1 oder 2, wobei das Lösungsmittelgemisch (I) oder das Lösungsmittelgemisch (II) oder das Lösungsmittelgemisch (I) und das Lösungsmittelgemisch (II) mindestens 80 Gew.-% Wasser enthält, jeweils bezogen auf das gesamte Lösungsmittelgemisch (I) oder (II).

4. Verfahren nach einem der Ansprüche 1 bis 3, wobei das Lösungsmittelgemisch (I) oder das Lösungsmittelgemisch (II) oder das Lösungsmittelgemisch (I) und das Lösungsmittelgemisch (II) weniger als 10 Gew.-% Salze enthält, jeweils bezogen auf das gesamte Lösungsmittelgemisch (I) oder (II).

5. Verfahren nach einem der Ansprüche 1 bis 4, wobei der pH-Wert des Lösungsmittelgemischs (I) oder der pH-Wert des Lösungsmittelgemischs (II) oder der pH-Wert des Lösungsmittelgemischs (I) und der pH-Wert des Lösungsmittelgemischs (II) im Bereich von 6,0 bis 7,0 liegt.

6. Verfahren nach einem der Ansprüche 1 bis 5, wobei das Lösungsmittelgemisch (I') zumindest teilweise als Lösungsmittelgemisch (I) in das Verfahren zurückgeführt wird, oder
wobei das Lösungsmittelgemisch (II') zumindest teilweise als Lösungsmittelgemisch (II) in das Verfahren zurückgeführt wird, oder
wobei das Lösungsmittelgemisch (I') zumindest teilweise als Lösungsmittelgemisch (I) in das Verfahren zurückgeführt wird und das Lösungsmittelgemisch (II') zumindest teilweise als Lösungsmittelgemisch (II) in das Verfahren zurückgeführt wird.

7. Verfahren nach einem der Ansprüche 1 bis 6, wobei der pH-Wert des Lösungsmittelgemischs (I) oder der pH-Wert des Lösungsmittelgemischs (II) oder der pH-Wert des Lösungsmittelgemischs (I) und der pH-Wert des Lösungsmittelgemischs (II) durch Zugabe einer Base ausgewählt aus der Gruppe bestehend aus Alkalimetallhydroxiden, Alkalimetallcarbonaten, Alkalimetallhydrogencarbonaten, Alkalimetallphosphaten, Erdalkalimetallhydroxiden, Erdalkalimetallcarbonaten, Erdalkalimetallhydrogencarbonaten und Erdalkalimetallphosphaten eingestellt wird.

8. Verfahren nach einem der Ansprüche 1 bis 7, wobei der Druck bei dem Inkontaktbringen gemäß Schritt A1 oder B1 oder A1 und B1 in einem Bereich von 10 bis 35 bar liegt.

9. Verfahren nach einem der Ansprüche 1 bis 8, wobei die Schritte A1 und A2 oder die Schritte B1 und B2 oder die Schritte A1 und A2 und die Schritte B1 und B2 in einer Trennwandkolonne durchgeführt werden.

10. Verfahren nach einem der Ansprüche 1 bis 9, wobei das erhaltene Gasgemisch G-2 verflüssigt wird.

11. Verfahren zur Herstellung eines Ketons mindestens umfassend die folgenden Schritte:
A1 Inkontaktbringen eines Gasgemisches G-0 enthaltend Distickstoffmonoxid und NOₓ mit einem Lösungsmittelgemisch (I) mindestens enthaltend 50 Gew.-% Wasser bezogen auf das gesamte Lösungsmittelgemisch (I), wobei der pH-Wert des Lösungsmittelgemischs (I) im Bereich von 3,5 bis 8,0 liegt, unter Erhalt einer Zusammensetzung (A);
A2 Desorption eines Gasgemisches G-1 aus der Zusammensetzung (A) unter Erhalt eines Lösungsmittelgemischs (I');
B1 Inkontaktbringen des Gasgemisches G-1 mit einem Lösungsmittelgemisch (II) mindestens enthaltend 50 Gew.-% Wasser bezogen auf das gesamte Lösungsmittelgemisch (II), wobei der pH-Wert des Lösungsmittelgemischs (II) im Bereich von 2,0 bis 8,0 liegt, unter Erhalt einer Zusammensetzung (B);
B2 Desorption eines Gasgemisches G-2 aus der Zusammensetzung (B) unter Erhalt eines Lösungsmittelgemischs (II');
C Inkontaktbringen des Gasgemisches G-2 mit mindestens einem Olefin;
wobei sich der pH-Wert jeweils auf eine Messung mit einer Glaselektrode bezieht,
wobei der pH-Wert des Lösungsmittelgemischs (I) oder der pH-Wert des Lösungsmittelgemischs (II) oder der pH-Wert des Lösungsmittelgemischs (I) und der pH-Wert des Lösungsmittelgemischs (II) durch Zugabe einer Base eingestellt wird.

## Claims

1. A process for purifying a gas mixture G-0 comprising dinitrogen monoxide and NOₓ, at least comprising the following steps:
A1 contacting the gas mixture G-0 with a solvent mixture (I) at least comprising 50% by weight of water based on the overall solvent mixture (I), the pH of the solvent mixture (I) being in the range from 3.5 to 8.0, to obtain a composition (A);
A2 desorption of a gas mixture G-1 from the composition (A) to obtain a solvent mixture (I');
B1 contacting the gas mixture G-1 with a solvent mixture (II) at least comprising 50% by weight of water based on the overall solvent mixture (II), the pH of the solvent mixture (II) being in the range from 2.0 to 8.0, to obtain a composition (B);
B2 desorption of a gas mixture G-2 from the composition (B) to obtain a solvent mixture (II');
the pH being based in each case on a measurement with a glass electrode,
wherein the pH of the solvent mixture (I) or the pH of the solvent mixture (II) or the pH of the solvent mixture (I) and the pH of the solvent mixture (II) is adjusted by adding a base.

2. The process according to claim 1, wherein the gas mixture G-0 is the offgas of an adipic acid plant and/or of a dodecanedicarboxylic acid plant and/or of a hydroxylamine plant and/or of a nitric acid plant operated with the offgas of an adipic acid plant and/or of a dodecanedicarboxylic acid plant and/or of a hydroxylamine plant.

3. The process according to either of claims 1 and 2, wherein the solvent mixture (I) or the solvent mixture (II) or the solvent mixture (I) and the solvent mixture (II) comprises at least 80% by weight of water, based in each case on the overall solvent mixture (I) or (II).

4. The process according to any of claims 1 to 3, wherein the solvent mixture (I) or the solvent mixture (II) or the solvent mixture (I) and the solvent mixture (II) comprises less than 10% by weight of salts, based in each case on the overall solvent mixture (I) or (II).

5. The process according to any of claims 1 to 4, wherein the pH of the solvent mixture (I) or the pH of the solvent mixture (II) or the pH of the solvent mixture (I) and the pH of the solvent mixture (II) is in the range from 6.0 to 7.0.

6. The process according to any of claims 1 to 5, wherein the solvent mixture (I') is recycled into the process at least partly as the solvent mixture (I), or
wherein the solvent mixture (II') is recycled into the process at least partly as the solvent mixture (II), or
wherein the solvent mixture (I') is recycled into the process at least partly as the solvent mixture (I) and the solvent mixture (II') is recycled into the process at least partly as the solvent mixture (II).

7. The process according to any of claims 1 to 6, wherein the pH of the solvent mixture (I) or the pH of the solvent mixture (II) or the pH of the solvent mixture (I) and the pH of the solvent mixture (II) is adjusted by adding a base selected from the group consisting of alkali metal hydroxides, alkali metal carbonates, alkali metal hydrogencarbonates, alkali metal phosphates, alkaline earth metal hydroxides, alkaline earth metal carbonates, alkaline earth metal hydrogencarbonates and alkaline earth metal phosphates.

8. The process according to any of claims 1 to 7, wherein the pressure in the contacting in A1 or B1 or A1 and B1 is in a range of from 10 to 35 bar.

9. The process according to any of claims 1 to 8, wherein steps A1 and A2 or steps B1 and B2 or steps A1 and A2 and steps B1 and B2 are carried out in a dividing wall column.

10. The process according to any of claims 1 to 9, wherein the resulting gas mixture G-2 is liquefied.

11. Process for preparing a ketone at least comprising the following steps:
A1 contacting a gas mixture G-0 comprising dinitrogen monoxide and NOₓ with a solvent mixture (I) at least comprising 50% by weight of water based on the overall solvent mixture (I), the pH of the solvent mixture (I) being in the range from 3.5 to 8.0, to obtain a composition (A);
A2 desorption of a gas mixture G-1 from the composition (A) to obtain a solvent mixture (I');
B1 contacting the gas mixture G-1 with a solvent mixture (II) at least comprising 50% by weight of water based on the overall solvent mixture (II), the pH of the solvent mixture (II) being in the range from 2.0 to 8.0, to obtain a composition (B);
B2 desorption of a gas mixture G-2 from the composition (B) to obtain a solvent mixture (II');
C contacting the gas mixture G-2 with at least one olefin;
the pH being based in each case on a measurement with a glass electrode,
wherein the pH of the solvent mixture (I) or the pH of the solvent mixture (II) or the pH of the solvent mixture (I) and the pH of the solvent mixture (II) is adjusted by adding a base.

## Revendications

1. Procédé de purification d'un mélange de gaz G-0 contenant de l'oxyde nitreux et du NOₓ, comprenant au moins les étapes suivantes :
A1 mise en contact du mélange gazeux G-0 avec un mélange de solvants (I) contenant au moins 50% en poids d'eau par rapport au mélange total de solvants (I), le pH du mélange de solvants (I) se situant dans la plage de 3,5 à 8,0, avec obtention d'une composition (A) ;
A2 désorption d'un mélange gazeux G-1 de la composition (A) avec obtention d'un mélange de solvants (I');
B1 mise en contact du mélange gazeux G-1 avec un mélange de solvants (II) contenant au moins 50% en poids d'eau par rapport au mélange total de solvants (II), le pH du mélange de solvants (II) se situant dans la plage de 2,0 à 8,0, avec obtention d'une composition (B) ;
B2 désorption d'un mélange gazeux G-2 de la composition (B) avec obtention d'un mélange de solvants (II');
le pH se rapportant à chaque fois à une mesure avec une électrode de verre,
le pH du mélange de solvants (I) ou le pH du mélange de solvants (II) ou le pH du mélange de solvants (I) et le pH du mélange de solvants (II) étant réglé par l'addition d'une base.

2. Procédé selon la revendication 1, le mélange de gaz G-0 étant le gaz résiduel d'une unité de production d'acide adipique et/ou d'une unité de production d'acide dodécanedioïque et/ou d'une unité de production d'hydroxylamine et/ou d'une unité de production d'acide nitrique exploitée avec le gaz résiduel d'une unité de production d'acide adipique et/ou d'une unité de production d'acide dodécanedioïque et/ou d'une unité de production d'hydroxylamine.

3. Procédé selon l'une quelconque des revendications 1 ou 2, le mélange de solvants (I) ou le mélange de solvants (II) ou le mélange de solvants (I) et le mélange de solvants (II) contenant au moins 80% en poids d'eau, à chaque fois par rapport au mélange total de solvants (I) ou (II).

4. Procédé selon l'une quelconque des revendications 1 à 3, le mélange de solvants (I) ou le mélange de solvants (II) ou le mélange de solvants (I) et le mélange de solvants (II) contenant moins de 10% en poids de sels, à chaque fois par rapport au mélange total de solvants (I) ou (II).

5. Procédé selon l'une quelconque des revendications 1 à 4, le pH du mélange de solvants (I) ou le pH du mélange de solvants (II) ou le pH du mélange de solvants (I) et le pH du mélange de solvants (II) se situant dans la plage de 6,0 à 7,0.

6. Procédé selon l'une quelconque des revendications 1 à 5, le mélange de solvants (I') étant recyclé au moins partiellement en tant que mélange de solvants (I) dans le procédé, ou
le mélange de solvants (II') étant recyclé au moins partiellement en tant que mélange de solvants (II) dans le procédé, ou
le mélange de solvants (I') étant recyclé au moins partiellement en tant que mélange de solvants (I) dans le procédé et le mélange de solvants (II') étant recyclé au moins partiellement en tant que mélange de solvants (II) dans le procédé.

7. Procédé selon l'une quelconque des revendications 1 à 6, le pH du mélange de solvants (I) ou le pH du mélange de solvants (II) ou le pH du mélange de solvants (I) et le pH du mélange de solvants (II) étant réglé par l'addition d'une base choisie dans le groupe constitué par les hydroxydes de métal alcalin, les carbonates de métal alcalin, les hydrogénocarbonates de métal alcalin, les phosphates de métal alcalin, les hydroxydes de métal alcalino-terreux, les carbonates de métal alcalino-terreux, les hydrogénocarbonates de métal alcalino-terreux et les phosphates de métal alcalino-terreux.

8. Procédé selon l'une quelconque des revendications 1 à 7, la pression, lors de la mise en contact selon l'étape A1 ou B1 ou A1 et B1, se situant dans une plage de 10 à 35 bars.

9. Procédé selon l'une quelconque des revendications 1 à 8, les étapes A1 et A2 ou les étapes B1 et B2 ou les étapes A1 et A2 et les étapes B1 et B2 étant réalisées dans une colonne à paroi de séparation.

10. Procédé selon l'une quelconque des revendications 1 à 9, le mélange de gaz G-2 obtenu étant liquéfié.

11. Procédé pour la préparation d'une cétone, comprenant au moins les étapes suivantes :
A1 mise en contact du mélange gazeux G-0 contenant de l'oxyde nitreux et du NOₓ avec un mélange de solvants (I) contenant au moins 50% en poids d'eau par rapport au mélange total de solvants (I), le pH du mélange de solvants (I) se situant dans la plage de 3,5 à 8,0, avec obtention d'une composition (A) ;
A2 désorption d'un mélange gazeux G-1 de la composition (A) avec obtention d'un mélange de solvants (I') ;
B1 mise en contact du mélange gazeux G-1 avec un mélange de solvants (II) contenant au moins 50% en poids d'eau par rapport au mélange total de solvants (II), le pH du mélange de solvants (II) se situant dans la plage de 2,0 à 8,0, avec obtention d'une composition (B) ;
B2 désorption d'un mélange gazeux G-2 de la composition (B) avec obtention d'un mélange de solvants (II') ;
C mise en contact du mélange de gaz G-2 avec au moins une oléfine ;
le pH se rapportant à chaque fois à une mesure avec une électrode de verre,
le pH du mélange de solvants (I) ou le pH du mélange de solvants (II) ou le pH du mélange de solvants (I) et le pH du mélange de solvants (II) étant réglé par l'addition d'une base.
